(19) Europäisches Patentamt
European Patent Office
Office européen des brevets

(11) Publication number: 0 133 795
B1

(12) **EUROPEAN PATENT SPECIFICATION**

(45) Date of publication of patent specification: **18.01.89**

(21) Application number: **84305226.7**

(22) Date of filing: **01.08.84**

(51) Int. Cl.$^4$: **C 07 C 101/18,**
C 07 C 101/28,
A 61 K 31/575, A 61 K 31/57,
A 61 K 31/70, C 07 J 41/00,
A 61 K 31/22, C 07 H 9/04,
C 07 H 13/04 // C07C125/065,
C07D317/24

(54) **Gaba esters and gaba analogue esters.**

(30) Priority: **01.08.83 US 519361**
**24.07.84 US 640507**

(43) Date of publication of application:
**06.03.85 Bulletin 85/10**

(45) Publication of the grant of the patent:
**18.01.89 Bulletin 89/03**

(84) Designated Contracting States:
**AT BE CH DE FR GB IT LI LU NL SE**

(56) References cited:

**CHEMICAL ABSTRACTS, vol. 85, no. 15, October 11, 1976, abstract 108263g, page 427 Columbus, Ohio, US "Synthesis of the derivatives of gamma-aminobutyric acid and lipids. II. Synthesis of 1,3-bis-O-(gamma-aminobutyryl)glycerol and 1,2,3-tris-O-(gamma-aminobutyryl) glycerol"**

(73) Proprietor: **The McLean Hospital Corporation**
**McLean Hospital**
**Belmont Massachusetts 02178 (US)**

(72) Inventor: **Shashoua, Victor E.**
**176, Tappan Street**
**Brookline, MA 02146 (US)**

(74) Representative: **Clifford, Frederick Alan et al**
**MARKS & CLERK 57/60 Lincoln's Inn Fields**
**London WC2A 3LS (GB)**

(56) References cited:

**CHEMICAL ABSTRACTS, vol. 84, no. 5, February 2, 1976, page 403, abstract 30348w Columbus, Ohio, US L.M. SHMUILLOVICH et al.: "Derivatives of gamma-aminobutyric acid and lipids. I. Synthesis of 1-O-(gamma-aminobutyryl)glycerol and 1,2-di-O-(gamma-aminobutyryl)glycerol**

**EP 0 133 795 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:

CHEMICAL ABSTRACTS, vol. 79, no. 13, October 1, 1973, page 29, abstract 73657k Columbus, Ohio, US R.U. OSTROVSKAYA et al.: "Analysis of the neurotropic activity of the cetyl ester of gamma-aminobutyric acid"

J. MED. CHEM. vol. 27, no. 5, 1984, Washington D.C., US American Chemical Society V.E. SHASHOUA et al.: "Gamma-Amino-butyric Acid Esters. 1. Synthesis, Brain Uptake, and Pharmacological Studies of Aliphatic and Steroid Esters of gamma-Aminobutyric Acid" pages 659-664

**Description**

Technical Field

The present invention relates to derivatives of gamma-aminobutyric acid (GABA) and to derivatives of gamma-aminobutyric acid analogues (GABA analogues), more particularly to ester derivatives of GABA and GABA analogues which cross the blood brain barrier, as well as to methods of synthesizing and using these compounds.

Background Art

GABA is an amino acid which has a ubiquitous distribution in the central nervous system (CNS) and fulfills the main criteria established for the identification of a neurotransmitter:

1 — GABA is synthesized and selectively released from nerve terminals,

2 — the release of GABA can be induced *in vitro* and *in vivo*,

3 — exogenously-applied GABA mimics the inhibitory effects elicited after neuronal stimulation,

4 — pre- and post-synaptic GABA receptors have been identified, and

5 — membrane transport systems and processes for determination of the neurotransmission process and for the inactivation of GABA, have been characterized.

The variation of CNS levels of GABA has been linked to certain neurological and psychiatric diseases. Low levels of GABA and of the GABA-synthesizing enzyme glutamate decarboxylase have been measured in post mortem brain tissue from patients suffering from Huntington's chorea. In Parkinson's disease, there is an imbalance between the GABA and the dopamine systems. In some brain areas of Parkinsonian patients, GABA receptor density is below normal levels. Analysis of brain samples from sites near seizure foci in epileptics revealed reduced GABA uptake capacity, probably reflecting degeneration of GABA neurons. Further, decreased GABA activity found in autopsies of schizophrenics suggests that GABA is also involved in the pathophysiology of this disease. Moreover, diseases where GABA neurons are still functioning, but at abnormally low levels, also lead to the postulation of a role for GABA in the etiology of CNS pathologies.

Under physiological conditions GABA does not significantly cross the blood-brain barrier. Thus, only at highly toxic doses was GABA shown in clinical studies to have a definite therapeutic action in epileptics, *Tower, D. B.,* in Nervous System Function: 461, Roberts et al, editors, Raven Press, N.Y., (1976). Consequently, clinically useful GABA agonists or prodrugs with a capacity to cross the blood-brain barrier would have extreme therapeutic relevance for the treatment of these conditions. Such an approach requires that GABA receptors on post-synaptic sites remain intact following the degeneration of GABA neurons.

Along these lines, several attempts have been made to increase GABAergic activity in the brain by administration of GABA derivatives or analogues.

Research by *Galzigna et al,* Arch. Int. Pharmacodyn. 235, 73 (1978), indicates that when the $NH_2$ function of GABA was blocked with either a benzoyl group, or a pivaloyl group, the resulting compounds were able to penetrate the blood-brain barrier when subcutaneously injected into rats.

Of the putative GABA mimetics which enter the brain, only muscimol has been widely examined, and even this compound enters the brain to a very limited extent, *Maggi et al,* J. Neuropharmacology 18, 361 (1979). However, muscimol exhibits an unacceptable CNS toxicity, which prevents its wide clinical use.

*Kaplan et al,* J. Med. Chem. 23, 702 (1980), have reported the use of derivatives where GABA is attached through an imine link (Schiff base) to a lipophilic carrier. The data show a reversal of bicuculline-induced lethality and convulsions indicating that these compounds can cross the blood-brain barrier of the rat.

*Krogsgaard-Larsen,* J. Med. Chem. 24 (12), 1377 (1981), reviewed the use of GABA agonists, antagonists and uptake inhibitors. Baclofen and cyclic GABA structures such as muscimol and its derivatives, Kojic amine, isoguvacine, nipecotic acid and its derivatives, among others are analysed, as well as their pharmacological activities in a variety of disorders. Of all the compounds analyzed, tetrahydroisoxazolopyridine-3-ol (THIP) penetrates the bloodbrain barrier without being peripherally degraded.

Chemical Abstracts, vol. 85, no. 15, abstract 108263g mentions mono-, di- and tri-ester forms of GABA-glycerol and the synthesis of the same. The utility of these compounds and their ability to pass the blood-brain barrier is neither discussed nor implied.

Chemical Abstracts, vol. 84, no. 5, abstract 30348w mentions mono- and di-ester forms of GABA-glycerol and the synthesis of the same. As with the previous reference, the utility of these compounds and their ability to pass the blood-brain barrier is neither discussed nor implied.

Chemical Abstract, vol. 79, no. 13, abstract 7365k mentions the neurotropic activity of the cetyl GABA ester. The ester is said to penetrate the blood-brain barrier. The subject matter of this reference is similar to that of the Frey et al prior art discussed in more detail below.

J. Med. Chem. (1948) vol 27, pages 659—664 discusses synthesis, brain uptake and pharmacological studies of aliphatic and steroid esters of GABA carried out by the applicant.

*Frey et al.* Neuropharmacology 19, 217 (1980), have shown that cetyl GABA has low anticonvulsant activity at dosages of 10—25 mg per kg when given intraperitoneally to mice and up to 100 mg per kg when given orally. The anticonvulsant effect was demonstrated only by threshold determinations, but there was

substantially no protection against seizures elicited by high doses of pentrazole or by the maximal electroshock, and, in both cases, there was substantially no effect on the extensor phase of the penetrazole convulsion. In addition to this low protection against convulsions and seizures, cetyl GABA was also shown to only slightly increase GABA levels in brain, thus suggesting that cetyl GABA may not reach the brain in substantially sufficient amounts. In addition, cetyl GABA was fairly toxic when given intravenously and intraperitoneally.

Thus, a number of agents having sufficient pharmacological activity have been tested, either as GABA agonists or antagonists, or for their ability to release GABA from the CNS neurons. At best, these approaches have resulted in a trade off between improvement of transport across the blood-brain barrier, and the worsening of side effects. Accordingly, new compounds capable of crossing the blood-brain barrier are needed.

Other GABA esters are also known in the prior art.

*Jones,* U.S. Patent No. 4,316,892, reported derivatives of N-enkephalin GABA to be useful as analgesics when administered orally in dosages of 0.5 to 5 mg per kg of body weight. These compounds include the free acid, lower alkyl esters, or alkylamide derivatives.

Somatostatin-GABA cyclic peptide derivatives having peripheral somatostatin-like activities have been reported by *Rink et al,* in U.S. Patent No. 4,328,214. These derivatives have a strong inhibiting effect on the insulin and glucagon secretion of the pancreas, and are therefore useful in the treatment of diabetes or blood losses in the gastrointestinal tract. Cyclic peptide derivatives with marked peripheral endocrine effect and substantially devoid of CNS activities are obtained by simple modifications of the basic peptidic structure, especially by omitting individual amino acids and/or exchanging them for other amino acids.

Certain analogues of GABA are known as well, *Johnson et al,* "GABA-Neurotransmitters," Alfred Benzon Symposium 12, Munksgaard (1978), and *Allan et al,* Medicinal Research Reviews, Vol. 3, No. 2, 91—118 (1983), incorporated by reference herein. Further, *Metcalf et al,* "GABA-Neurotransmitters," Alfred Benzon Symposium 12, Munksgaard (1978), identified two GABA analogues, gamma-acetylenic GABA and gamma vinyl GABA, as enzyme-activated inhibitors of GABA aminotransferase. The authors suggest that by inhibiting the GABA aminotransferase, elevated brain levels of GABA could be expected, perhaps having a beneficial effect on Huntington's disease, epilepsy and schizophrenia.

Accordingly, prior to the present invention there remained a great need for GABA and GABA analogue derivatives which could be transported across the blood-brain barrier, and which could be possibly hydrolyzed in the CNS to produce GABA and GABA analogues.

Disclosure of the Invention

It is therefore an object of the invention to provide ester derivatives of GABA and GABA analogues which cross the blood-brain barrier.

It is also an object of this invention to provide ester derivatives of GABA and GABA analogues having a Brain Penetration Index (BPI), as defined hereinafter, greater than about 2%.

Another object of the invention is to provide ester derivatives of GABA and GABA analogues having an n-octanol/water partition coefficient ($K_{OW}$), as defined hereinafter, greater than about 1.

It is another object of the invention to provide GABA and GABA analogue esters with biological activity useful in the regulation of general locomotor activity.

It is another object of the invention to provide GABA derivatives and GABA analogue derivatives with biological activity useful in the prevention and/or treatment of seizure.

It is yet another object of the invention to provide pharmacological compositions comprising esters of GABA and esters of GABA analogues.

It is still a further object of this invention to provide a general method of synthesizing ester derivatives of GABA and ester derivatives of GABA analogues.

An advantage of this invention is to provide a method of regulating general motor activity in animals, including humans, and to provide a method of preventing and/or treating seizures, epilepsy, Huntingdon's disease, manic depression, general depression, schizophrenia and/or neuropsychiatric disorders.

These and other objects of the invention, as will hereinafter become more readily apparent, have been attained by providing:

A compound of the formula:

$$A(O-CO-\underset{|}{\overset{R_3}{}}CH-\overset{R_2}{\underset{|}{}}CH-\overset{R_1}{\underset{|}{}}CH-\overset{R_4}{\underset{|}{}}NH)_n$$

wherein $R_1$, $R_2$ and $R_3$ are the same or different and are selected from:

(a) hydrogen

(b) lower alkyl groups having one to four carbon atoms;

(c) substituted lower alkyl groups having one to four carbon atoms;

(d) lower alkenyl groups having one to four carbon atoms;

(e) substituted lower alkenyl groups having one to four carbon atoms;

(f) lower alkynyl groups having one to four carbon atoms;

(g) substituted lower alkynyl groups having one to four carbon atoms;

(h) aryl groups;
(i) substituted aryl groups;
(j) hydroxy groups or hydroxy groups protected with lower acyl or aroyl groups;
(k) lower acyl groups;
(l) oxo groups, in which case hydrogen is not present on the carbon atom;
(m) amino groups;
(n) substituted amino groups;
(o) $R_1$ and $R_3$ together forming a carbocyclic ring;
(p) $R_2$ and $R_3$ together forming a carbocyclic ring;
(q) $R_4$ is hydrogen or acyl;

A represents the radical of a compound having at least one esterifiable OH group, selected from the group consisting of carbohydrates, saturated aliphatic alcohols with 4—40 carbon atoms having more than one OH substituent, unsaturated aliphatic alcohols, cyclic alcohols, aromatic alcohols, alcohols containing at least one heteroatom and glyceryl esters of fatty acids, and being capable of crossing the blood-brain barrier of an animal;

n can vary from 1 to the total number of esterifiable OH groups contained in A;
or pharmaceutically acceptable acid addition salts thereof,
said ester having a Brain Penetration Index greater than about 2%.

The objects have also been attained by providing pharmacological compositions comprising the aforementioned compounds.

A general method of synthesis for the compounds described above is also part of the present invention. This process comprises reacting GABA, or an anhydride of a GABA derivative with a compound having at least one esterifiable OH group. As esterification reaction takes place between the carboxy group of GABA and one of the hydroxy groups of the alcohol to form an ester derivative of GABA.

Further, the objects of the invention have also been attained by providing a method of promoting the uptake of GABA by the brain, and a method of treating a condition associated with abnormal GABA levels in the brain.

A method of preventing or treating seizures, epilepsy, Huntingdon's disease, manic depression general depression, schizophrenia and/or neuropsychiatric disorders is also herein provided.

Other objects, advantages and features of the present invention will become apparent to those skilled in the art from the following discussion.

Brief Description of the Drawings

Figures 1A and 1B show the effects of cholesteryl-GABA on general motor activity. Figure 1A shows the effects of the dose of the compound on the behavior of rats and mice (n = 6 per dose) and Figure 1B shows the time course of recovery from reduced activity of the animals at a dose of 21µ mol/kg (n = 6 per group).

Figure 2 shows the dose-response data for the effect of 1-linolenoyl-2,3-(4-aminobutyroyl) propane-1,2,3-triol (Compound *26*) on the general motor activity of test mice. The compound enhances motor activity at the low dose of 10 mg/kg (19 µmol/kg) and reduces activity at high doses ($ED_{50}$ = 36 µmol/kg (69 µmol/kg). The points show the mean ± S.D. for eight test animals at each dose.

Best Mode for Carying Out the Invention

By GABA and GABA analogues is intended compounds having the general formula (I):

$$R_4\text{-NH-}\overset{R_1}{\overset{|}{CH}}\text{-}\overset{R_2}{\overset{|}{CH}}\text{-}\overset{R_3}{\overset{|}{CH}}\text{-COOH} \qquad (I)$$

where $R_1$, $R_2$ and $R_3$ are the same or different and include, but are not limited to,
(1) hydrogen;
(2) lower alkyl groups having one to four carbon atoms;
(3) substituted lower alkyl groups having one to four carbon atoms;
(4) lower alkenyl groups having two to four carbon atoms;
(5) substituted lower alkenyl groups having two to four carbon atoms;
(6) lower alkynyl groups having two to four carbon atoms;
(7) lower substituted alkynyl groups having two to four carbon atoms;
(8) aryl groups;
(9) substituted aryl groups;
(10) hydroxygroups or protected hydroxy groups such as lower ($C_1$—$C_4$) acyl or aroyl groups;
(11) oxo groups, in which case, hydrogen is not present on the same carbon;
(12) amino groups;
(13) substituted amino groups;
(14) $R_1$ and $R_3$ together form a carbocyclic ring;
(15) $R_2$ and $R_3$ together form a carbocyclic ring.

Typical lower alkyl groups include methyl, ethyl, n-propyl, i-propyl, n-butyl, t-butyl and i-butyl. The preferred alkyl is methyl, the substitution preferably in the gamma position.

Typical substituted lower alkyl groups include alkyl groups having one to four carbon atoms and substituted with such groups as hydroxy, halo, amino, and the like. Typical substituted lower alkyl GABAs include gamma-fluoro methyl GABA and gamma-difluoro-methyl GABA.

Typical lower alkenyl groups include vinyl, allyl, and methylene, with vinyl preferred. Gamma-vinyl GABA is a preferred analogue.

Typical substituted lower alkenyl groups include the hydroxy, halo and amino substituted lower alkenyl groups.

Typical lower alkynyl groups are those lower alkynyl groups having two to four carbon atoms and include acetynyl and propargyl, with gamma-substituted lower alkynyl GABA's preferred.

Typical halo groups include fluorine, chlorine, bromine and iodine, with fluorine and chlorine preferred. Typical halo-substituted GABA's include 2- and 3-chloro-GABA and 3-fluoro-GABA.

Typical hydroxy substituted compounds include those compounds with hydroxy substituted in the alpha, beta, and gamma positions, with the alpha-hydroxy- and beta-hydroxy-GABA's preferred.

Typical aryl groups include phenyl, naphthyl, phenanthryl and anthracyl, with phenyl preferred.

Typical substituted aryl groups include aryls substituted with halo, hydroxy, amino, and the like. Halo substituted aryls are preferred, with chlorophenyl most preferred.

Typical amino groups include —$NHR_5$ where $R_5$ is hydrogen or a substituent such as hydroxy, lower alkyl, amino, or the like. Preferred amino groups are those wherein $R_5$ is hydrogen. Preferred amino-substituted GABA's are those where the amino group is substituted in the alpha position.

Typical oxo-substituted GABA's include those where the oxo group is substituted in the alpha, beta or gamma position, with beta-oxo-substituted GABA preferred.

Where $R_1$ and $R_3$ together form a carbocyclic ring, typical carbocyclic rings contain three to six carbon atoms. Typical carbocyclic rings are cyclopropane, cyclopentane, and cyclohexane, with cyclopentane the preferred carbocyclic ring. Where the carbocyclic ring contains five or six members, unsaturation in the ring is included as a part of the invention.

Where $R_2$ and $R_3$ taken together form a carbocyclic ring, typical carbocyclic rings include three to six-member carbocyclic rings, with cyclopropane preferred.

$R_4$ may be hydrogen or an acyl group where $R_4$ is acyl, preferred acyl groups include acetyl, pivaloyl, and benzoyl.

The geometric and stereoisomers of compounds having the general formula (I) are also within the scope of the present invention.

These compounds having the general formula (I) are well-known to the prior art and their synthesis within the skill of the prior art. See particularly *Allan et al, supra.*

By GABA analogue esters or esters of GABA analogues is intended compounds having the general formula (II):

$$A-(O-CO-\underset{\mid}{\overset{R_3}{C}}H-\underset{\mid}{\overset{R_2}{C}}H-\underset{\mid}{\overset{R_1}{C}}H-NH-R_4)_n \qquad (II)$$

where $R_1$, $R_2$, $R_3$ and $R_4$ are as described above and A represents the radical of a compound having at least one esterifiable OH group capable of crossing the blood-brain barrier of an animal, and n can vary from 1 to the total number of esterifiable OH groups contained in A. Moreover, pharmaceutically acceptable acid addition salts of the former compounds, are also a part of the present invention.

The compounds from which A is derived may be endogenous or exogenous to mammals. However, the extent to which these compounds have free access to the brain from the blood flow makes them useful for the preparation of the present GABA esters and GABA analogue esters. These compounds comprise a broad range of lipophilicities. While for some, crossing the blood-brain barrier is accomplished through an active transport system, other compounds are taken up by the brain passively by diffusion. The compounds of use in the present invention are those which when present in the blood flow can cross the blood-brain barrier to a substantial extent.

The criterion used herein for measuring the extent to which a compound, from which the radical A is derived, crosses the blood-brain barrier is based on the uptake of this compound by the brain relative to its uptake by the liver. The liver was chosen as a reference since it is an organ which has no barrier to diffusible molecules present in the blood. Measurements of the quantity of a compound present in brain and liver at 5 minutes after a subcutaneous (s.c.) injection were used to calculate a Brain Penetration Index (BPI), with the equation:

$$BPI = \frac{(brain)}{(blood)} \times 100$$

This is the amount of the compound present per gram of brain tissue as a percent of the amount per gram of liver tissue.

This procedure has the advantage that even for a sparingly soluble lipid ester, which tends to remain largely at the site of the injection and slowly diffuse into the circulation, its level in the liver will reflect the amount available rather than the initial dose injected.

Some of the preferred compounds from which the radical A is derived are those having a BPI of between about 2 and 500%.

Some of the most preferred compounds have a BPI of between about 3 and 300%; and still more preferred are those compounds having a BPI of between 25 and 200%.

A may be selected from the group consisting of compounds derived from carbohydrates, saturated aliphatic alcohols having more than one OH substituent, unsaturated aliphatic alcohols and their derivatives containing one or more heteroatoms, saturated and unsaturated cyclic alcohols, heterocyclic alcohols, aromatic alcohols and their derivatives containing one or more heteroatoms, and glycerides which are unesterified or esterified with saturated and unsaturated aliphatic fatty acids. Also included is dihydroxyacetone.

The ester derivatives of GABA and GABA analogues may be in the form of the free amines (on the N-terminus) or acid addition salts thereof. Acid addition salts may be, e.g., hydrohalic acid salts, for example salts of HBr, HF, or more preferably, HCl.

Preferred are those GABA and GABA analogue esters having a Brain Penetration Index (BPI), as defined hereinbefore, greater than about 2%. Among this group, preferred are those compounds which have a BPI greater than about 3%; but most preferred yet, are those GABA and GABA analogue esters having a BPI greater than about 25%.

Also preferred among the esters of GABA and GABA analogues are those wherein A is the radical of a carbohydrate having a carbon chain of $C_3$—$C_{20}$ carbon atoms, their O-acetyl derivatives, their O-methyl derivatives, their deoxy-D-derivatives, their amino derivatives and their acid derivatives.

Among the most preferred carbohydrates are those having a carbon chain of 3 to 7 carbon atoms.

Some of the most preferred, are carbohydrates of the formula:

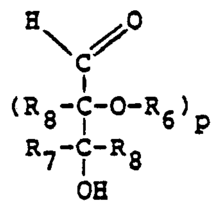

wherein:

(1) p is 1 to 18,

(2) $R_6$, $R_7$ and $R_8$ may be the same or different and represent:

(a) H;

(b) OH, except that $R_7$ and $R_8$ cannot be OH;

(c) branched or unbranched amino of the formula $(CH_2)_nNH_2$, wherein n is 1 to 15;

(d) branched or unbranched hydrazino of the formula $(CH_2)_nNHNH_2$, wherein n is as defined above;

(e) haloalkyl of the formula $(CH_2)_nCX_mH_{(3-m)}$, wherein n is as defined above and m is 1 to 3;

(f) alkyl of 1 to 15 carbon atoms;

(g) alkenyl of 1 to 15 carbon atoms;

(h) alkynyl of 1 to 15 carbon atoms; or

(i) $(CH_2)_nOPO_3H_2$, wherein n is as defined above; and in addition

(3) $R_7$ and $R_8$ may be the same or different and represent haloalkyl of the formula $CX_mH_{(3-m)}$ wherein m and X are as defined above;

(4) $R_6$ reprsents acetyl.

Representative GABA and GABA analogue esters of the present invention are those wherein A is a carbohydrate derived from D-glyceraldehyde, D-erythrose, D-threose, D-ribose, D-arabinose, D-xylose, D-allose, D-glucose, D-mannose, D-gulose, D-idose, D-galactose, D-talose, dihydroxy acetone, D-erythrulose, D-ribulose, D-xylulose, D-psikose, D-fructose, D-sorbose, their O-acetyl derivatives, their O-methyl derivatives, their corresponding deoxy-D-derivatives, their corresponding amino derivatives; and their corresponding acid derivatives.

Also preferred among the ester derivatives of GABA and GABA analogues are those wherein A is a radical or a branched or unbranched saturated aliphatic alcohol having a carbon chain of 18 to 40 carbon atoms. Some of the most preferred are those having an even number of carbon atoms, and those having a carbon chain containing 20 to 26 carbon atoms.

Preferred among the GABA and GABA analogue esters are also those wherein A is a radical derived from a branched or unbranched saturated aliphatic alcohol having a carbon chain with 2 to 40 carbon atoms. Some of the most preferred are those having an even number of carbon atoms, and those having a carbon chain containing 20 to 26 carbon atoms.

Among the most preferred alcohols are those having multiple hydroxyl substituents.

Representative ester derivatives of the present invention, wherein A represents a radical of a branched or unbranched saturated aliphatic polyalcohol are those derived from glycerol, n-butanol, isobutanol, sec-butanol, 1,2,3,4-tetrahydroxybutanol, n-pentanol, isopentanol, 1,2,3,4-tetrahydroxypentanol, n-hexanol, n-heptanol, n-octanol, n-decanol, n-dodecanol, n-tetradecanol, n-octadcanol, n-eicosanol, and n-tetracosanol.

Also preferred are the GABA and GABA analogue esters wherein A is the radical of an aliphatic alcohol having one or more points of unsaturation of a carbon chain consisting of 3 to 40 carbon atoms. Among these, preferred are thóse with a carbon chain having an even number of carbon atoms. Another group of preferred alcohols is alkynyl alcohols.

Another group of preferred compounds is that having aliphatic alcohols with one or more points of unsaturation and a carbon chain of 4 to 30 carbon atoms. Among the most preferred unsaturated alcohols are those having a carbon chain of 8 to 20 carbon atoms.

Some of the most preferred alcohols are the alkenyl and alkynyl alcohols with multiple points of unsaturation, and among these the most preferred ones are those with an even number of carbon atoms in the carbon chain.

Representative GABA and GABA analogue esters of the present invention, wherein A represents a radical of a branched or unbranched aliphatic alcohol having at least one point of unsaturation, are those derived from allyl alcohol, crotyl alcohol, methylvinylcarbonol, palmitolyl alcohol, oleic alcohol, linoleic alcohol, linolenic alcohol, arachidonic alcohol, lactobacillic alcohol, cerebronic alcohol, their alkyl derivatives of 1 to 10 carbons, their corresponding hydroxyl derivatives, their corresponding halo derivatives having F, Cl, Br or I, their corresponding amino derivatives, and their corresponding acid derivatives.

Preferred among the GABA and GABA analogue esters, are also those wherein A is the radical of a saturated or unsaturated cyclic alcohol with one or more points of unsaturation. Also preferred are those cyclic alcohols having one or more carbon rings with a maximum of 18 ring carbon atoms. Also preferred are those wherein one or more of the carbon rings is derived from alkenyl or alkynyl alcohols with multiple unsaturation points.

Some of the most preferred cyclic alcohols are those having 5 to 14 carbon ring atoms. Another group of preferred cyclic alcohols is that having 6 to 10 carbon atoms.

Most preferred among the cyclic alcohols are those having an even number of carbon atoms. Some of the most preferred cyclic alcohols are those having multiple hydroxyl substituents.

Representative GABA and GABA analogue esters of the present invention, wherein A represents the radical of a branched or unbranched, saturated or unsaturated cyclic alcohol, are those derived from cyclopentanol, cyclohexanol, cycloheptanol, cyclooctanol, cyclodecanol, cyclododecanol, cyclotetradecanol, cyclohexadecanol, cyclooctadecanol, inositol, their derivatives having multiple hydroxyl substituents, their O-acetyl derivatives, their O-methyl derivatives, their amine derivatives, and their halogenated derivatives.

Also representative GABA and GABA analogue esters of the present invention, wherein A represents a radical or a branched or unbranched, saturated or unsaturated cyclic alcohol, containing one or more unsaturated rings, are those derived from dexamethasone having the formula:

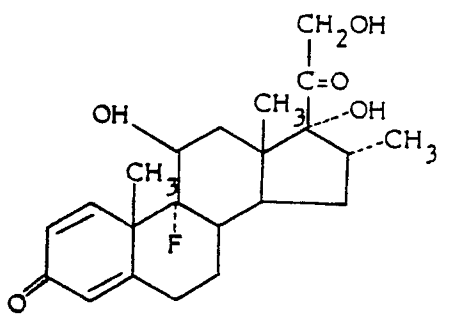

Also preferred among the GABA and GABA analogue esters of cyclic alcohols are those wherein A is the radical of an alcohol containing one or more aromatic carbon rings. Among some of the most preferred compounds are those having one unsaturated carbon ring and containing a maximum of 18 carbon ring atoms.

Also preferred are the alcohols selected from the group consisting of the aliphatic and alicyclic alkyl, alkenyl and alkynyl alcohols having one or more points of unsaturation and the aromatic alcohols of the formula:

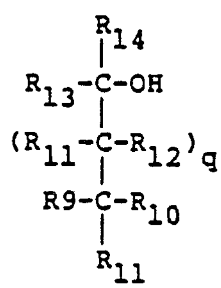

wherein:

(1) q is 1 to 38;

(2) $R_9$, $R_{10}$, $R_{11}$, $R_{12}$, $R_{13}$ and $R_{14}$ may be the same or different, and represent:

(a) H, with the proviso that at least one is different from H;

(b) OH, except that $R_{13}$ and $R_{14}$ are not OH;

(c) branched or unbranched groups of the formula $(CH_2)_nNH_2$, wherein n is 1 to 15;

(d) branched or unbranched alkyl groups of 1 to 15 carbon atoms;

(e) branched or unbranched hydrazino groups of the formula $(CH_2)_nNHNH_2$, wherein n is as defined above;

(f) branched or unbranched haloalkyl groups of the formula $(CH_2)_nCX_mH_{(3-m)}$, wherein

X represents F, Cl, Br, or I,

n is defined as above, and

m is 1 to 3;

(g) branched or unbranched alkenyl of 2 to 15 carbon atoms;

(h) branched or unbranched alkynyl of 2 to 15 carbon atoms;

(i) acetyl;

(j) their O-acetyl derivatives;

(k) their O-methyl derivatives; and,

if there is at least one carbon ring,

(3) $R_{10}$ and $R_{12}$ may be a cyclic structure containing one or more carbon rings.

Also preferred among the GABA and GABA analogue esters are those where A is the radical of an alcohol having one or more heteroatoms. Most preferred heteroalcohols are those having a carbon chain of 2 to 40 carbon atoms. Among the most preferred alcohols are those having a carbon chain with 6 to 30 carbon atoms. Among these, most preferred are those alcohols having a carbon chain with 10 to 26 carbon atoms.

Also preferred are those alcohols containing one or more nitrogen, sulfur or oxygen atoms, or a combination thereof.

Also preferred are those heteroalcohols having one or more carbon rings. Among these, the most preferred ones are those having 1 or more points of unsaturation in the hetero ring.

Still another group of preferred heteroalcohols are those having one or more aromatic rings.

Representative GABA and GABA analogue derivatives of the present invention, wherein A represents a radical of a branched or unbranched aliphatic or cyclic alcohol having one or more heterocyclic atoms, are those derived from ethanolamine, choline, serine, O-aminoacylglycerol, their alkyl derivatives of 1 to 15 carbon atoms, their O-methyl derivatives of 1 to 15 carbon atoms, their O-acyl-derivatives of 1 to 15 carbon atoms, and the corresponding haloacid addition salts.

Some of the preferred compounds of the present invention are those wherein A represents a radical of a glycerol esterified with from 1—2 radicals of carboxylic acids having a carbon chain with 2 to 60 carbon atoms. Among these, some of the most preferred are those compounds having carboxylicacids with an even number of carbon atoms. Another group of preferred carboxylic acids are fatty acids having a carbon chain of 6 to 40 carbon atoms and some of the most preferred among them are fatty acids with 10 to 30 chain carbon atoms. Another group of preferred compounds are those where the fatty acids have at least one point of unsaturation. And yet another group of preferred GABA and GABA analogue esters are those where the fatty acids have multiple points of unsaturation.

Further representative GABA and GABA analogue esters of the present invention, wherein A represents a radical of a glycerol esterified with from 1 to 2 radicals of fatty acids, which may be the same or different, are those derived from lauric acid, myristic acid, palmitic acid, stearic acid, arachidic acid, palmitoleic acid, oleic acid, linoleic acid, linolenic acid, arachidonic acid, cerebronic acid, cardiolypin, their O-acetyl derivatives, their O-methyl derivatives; and their amino derivatives.

Among the most preferred ester derivatives of GABA of this invention are butyl-GABA, butyl-gamma-vinyl GABA, linolenoyl-GABA, linolenoyl-gamma-vinyl GABA, dexamethasone-GABA, dexamethasone-gamma-vinyl-GABA, glyceryl-GABA, glyceryl-gamma-vinyl GABA, cholesterol-GABA, cholesterol-gamma-vinyl GABA, inositol-monoGABA, inositol-gamma-vinyl GABA, inositol-diGABA, inositol-di(gamma-vinyl GABA), inositol-triGABA, inositol-tri(gamma-vinyl GABA), diGABA-linolenoyl-glyceride, divinyl GABA-linolenoyl-glyceride, GABA-dilinolenoyl-glyceride, vinyl GABA-dilinolenoyl-glyceride and their acid addition salts.

Among the broad objectives of the present invention has been to develop a general method for the exogenous regulation of GABA and GABA analogue levels in brain. Given that GABA and GABA analogues are not taken up by the CNS in sufficient amounts, a replacement therapy approach was conceived based on the rationale of attaching GABA and GABA analogue to other compounds capable of penetrating the blood-brain barrier.

Thus, GABA, a substantially hydrophilic neurotransmitter, or a GABA analogue, is coupled to the radical A of a compound such as glucose, inositol and cholesterol, among others to be transported into the CNS. As in the case of the compound from which the radical A is derived, the criterion used herein for selecting the GABA or GABA analogue derivatives of the present invention is based on the extent of their uptake by the brain. Preferred GABA and GABA analogue derivatives are those which substantially

penetrate the brain, as measured by the BPI hereinbefore defined.

While the BPI value for GABA is about 1% (see Example 1), a compound for which there is substantially no blood-brain barrier has a BPI of about 100%, or equal uptake per gram of brain or liver. Compounds with BPI values substantially greater than 100% preferably accumulate in the brain and are also useful.

Some of the preferred compounds of this invention are those having a BPI of between about 2—500%. Some of the most preferred GABA and GABA analogue esters are those having a BPI of between about 3—300%, or 25 to 200%.

After crossing the blood-brain barrier, the compounds of the present invention act as agonists, partial agonists, antagonists and inhibitors of CNS-mediated activity. These GABA and GABA analogue esters act at the pre- and post-synaptic neurons, at membrane sites and at the different GABA receptor sites, as well as acting, in some cases, as inhibitors of the enzymes which catabolize GABA.

Additionally, since the attachment of these compounds to GABA and GABA analogues is through an ester bond, these derivatives act as prodrugs which may be hydrolyzed *in situ* providing a means to increase GABA and GABA analogue levels in the CNS. Given the different characteristics of the groups attached to GABA and GABA analogues, the rate of hydrolysis will be different for different GABA and GABA analogue ester derivatives. Thus, shorter and longer acting compounds, having GABAergic activities, are produced.

Some of the GABA and GABA analogue derivatives preferentially locate in specific parts of the brain tissue. Thus, compounds with different radicals have different biological activities which are also different from those of GABA or the GABA analogue itself. Such compounds are useful as dissecting tools of the neuroanatomy of the different GABA receptors in brain and in the treatment of different conditions. The latter effect is a consequence of the fact that while a GABA receptor present in one area of the brain causes, e.g., a decrease in locomotor activity, the same receptor present in another circuit in a different area of the brain causes, e.g., enhanced locomotor activity in an animal.

Since a significant proportion of the brain is composed of extracellular space and some of the present compounds are substantially water-soluble they are thus capable of acting at receptors located therein. Other compounds which are more lipid-soluble are capable of interacting with receptors located in the lipid areas of the brain. These compounds show a biological activity related to the receptors located in more lipidic tissues.

Some of the preferred compounds accumulate preferentially in the CNS after administration and, thus, their presence in brain tissues increases with time. The storage of lipid-soluble compounds in lipophilic-tissues is well known. The more lipophilic GABA esters are stored in the brain and are slowly released from their lipid tissue storage. Due to the similarity of some of the compounds to natural components of membrane lipids, some of the GABA and GABA analogue derivatives can also become associated with brain membrane lipid bilayers. The storage of these GABA or GABA analogue esters provides a reservoir for the release of GABA or GABA analogue by hydrolysis by the esterases present in the cerebrospinal fluid (CSF) and brain membrane.

Additionally, some of the GABA and GABA analogue compounds show a multiple-phase response. This is the case, e.g., of monolinolenoyl-diGABA glyceride, which has a stimulating activity at low doses, while behaving as a sedative at high doses.

The criterion used herein for selecting those GABA and GABA analogue esters having biological activity when administered to an animal is based on their differential lipid-water solubilities. A Partition Coefficient was defined by the equation:

$$K_{OW} = \frac{(\text{n-Octanol})}{(H_2O)}$$

representing the amount of the ester taken up per ml of n-octanol relative to the amount of the same compound taken up per ml of water.

Compounds having significantly low values of $K_{OW}$ have not yet been shown to have biological activity. GABA itself has a $K_{OW}$ of about 0.004 and is inactive when administered to an animal. A compound having a $K_{OW}$ of about 1 will be as soluble in octanol as in water.

Typical GABA and GABA analogue esters have a $K_{OW}$ of between about 0.1—10,000. Preferred compounds are those that having $K_{OW}$ of between about 1—5,000. A further group of preferred compounds is that having a $K_{OW}$ of between about 2—1,000. Some of the most preferred GABA and GABA analogue esters have a $K_{OW}$ of between 6—600.

Compounds of the invention can be synthesized by any general method of ester synthesis now known or discovered in the future. However, as has been previously mentioned, this invention also encompasses a general method of synthesis of ester derivatives of GABA and GABA analogues. This reaction is shown schematically, hereinafter, as Schemes I, II, III and IV.

Some of the compounds can be synthesized by simple esterification of GABA or a GABA analogue and the corresponding alcohol in the presence of a strong acid, as is the case for the butyl ester of GABA or GABA analogue shown in Scheme I.

SCHEME I

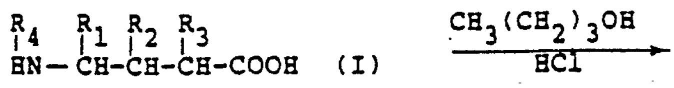


GABA or GABA Analogue

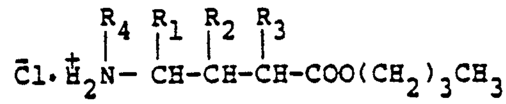

Butyl GABA or Butyl GABA Analogue
(III)

The preferred strategy for the synthesis of compounds having several OH groups, such as glyceryl derivatives and polyalcohols such as inositol or different carbohydrates, includes the protection of the various OH groups not to be esterified, by forming an acetonide derivative as shown in Scheme II, below. When reacting a hydroxy GABA analogue, it may also be necessary to protect the hydroxy group or groups. Appropriate protecting groups include lower ($C_1$—$C_4$ acyl or lower substituted acyl, e.g.

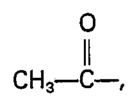,

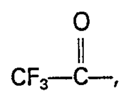,

or aroyl, e.g.

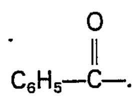.

SCHEME II

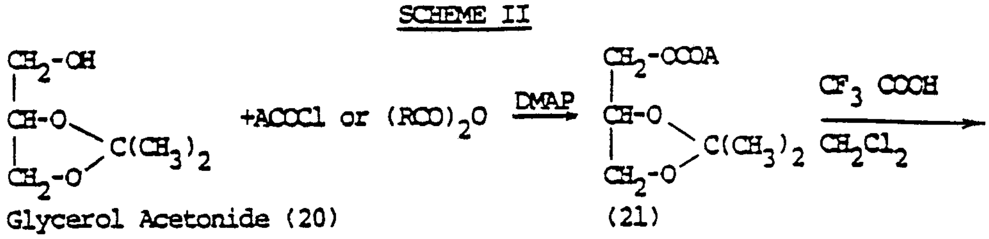


Glycerol Acetonide (20)

(21)

[DMAP: 4-dimethylaminopyridine]

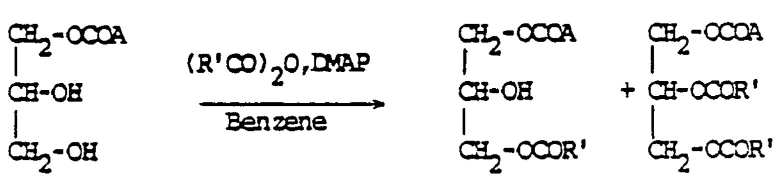


(22)

(23)

(24)

1-Glyceryl-linolenoate

A: $CH_3(CH_2CH{=}CH)_3(CH_2)_7$

R': 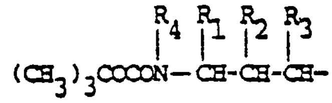

(23) 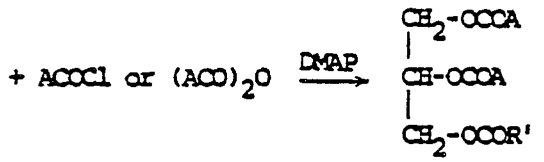


Triglyceride Ester (25)

(24) 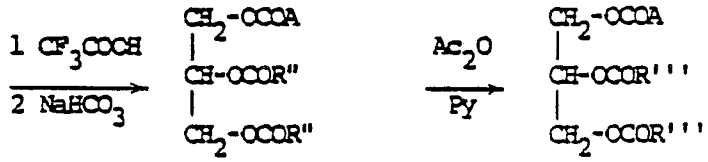


(26) (27)

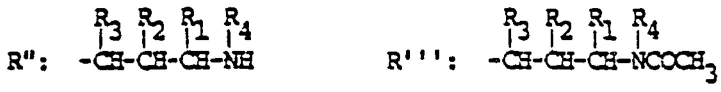


(25) 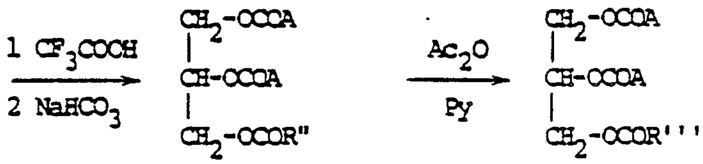


This general scheme also applies to the synthesis of mono, di, tri, and multiple GABA and GABA analogue derivatives of compounds such as inositol or different carbohydrates.

In the case of the glyceryl derivatives, two of the hydroxyl groups of glycerol can be protected by forming an acetonide, then the remaining hydroxyl group can be esterified with the symmetrical anhydride of a fatty acid in the presence of, for instance 4-dimethylaminopyridine, in a dry organic solvent to give the corresponding ester plus one mole of the fatty acid. This product after purification, e.g., chromatography on silica gel, can be next treated with a strong acid, e.g., trifluoroacetic acid, at 0—5°C in an organic solvent, e.g., methylene chloride, to give the diol ester. This compound can be separated again, e.g., on a silica gel column, from the unconverted starting material.

Further treatment of the mono ester of glycerol, with a GABA or GABA analogue anhydride having the amino terminus protected, e.g., with a t-butoxycarbonyl radical, in the presence of, for example, 4-dimethylaminopyridine, and an organic solvent, e.g., benzene, gives a mixture of compounds having one and two GABA or GABA analogue ester bonds in addition to the fatty acid ester bond. Different molar equivalent amounts of the GABA or GABA analogue anhydride reagent can be used to increase the amount obtained of one or the other. These mono and di GABA or GABA analogue ester derivatives can be separated from the starting materials, e.g., by chromatography on silica gel.

Further treatment of the diester derivative having a free hydroxyl group with the anhydride of chloride derivative of a fatty acid in the presence of, e.g., 4-dimethylaminopyridine, yields the corresponding triester with only one GABA or GABA analogue. All three mono, di and tri esters thus obtained can be converted to their free amino derivatives by treatment with trifluoroacetic acid at 0—5°C under a nitrogen atmosphere to remove the protecting groups. The trifluoroacetate salts formed after this step can be converted to the free amines by extraction of chloroform solutions of these compounds with dilute aqueous sodium bicarbonate. The free amines and the intermediates are fairly stable when stored in chloroform solution below 0°C.

The mono-GABA or mono-GABA analogue ester of glycerol can be synthesized by reacting the monoacetonide of glycerol with N-carbobenzoxy-GABA anhydride or N-carbobenzoxy-GABA analogue anhydride to obtain the corresponding ester. This product can be converted to glyceryl GABA or glyceryl GABA analogue by hydrogenolysis and treatment with trifluoroacetic acid. This route is shown in general in Scheme III.

As is well understood by those with ordinary skill in the art, similar blocking techniques may be utilized to protect reactive substituents along the carbon chain of the GABA analogue precursors as well, for example, where $R_1$, $R_2$ or $R_3$ is a hydroxy group.

SCHEME III

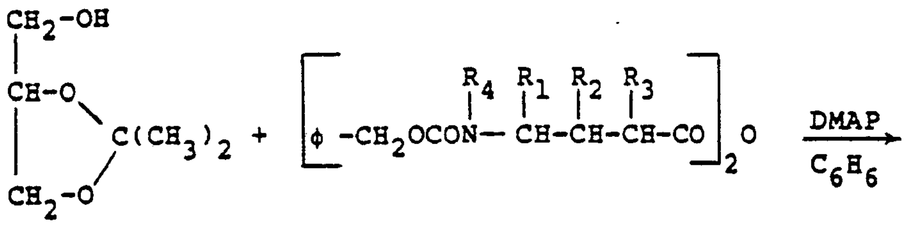


Glycerol-Acetonide (20)

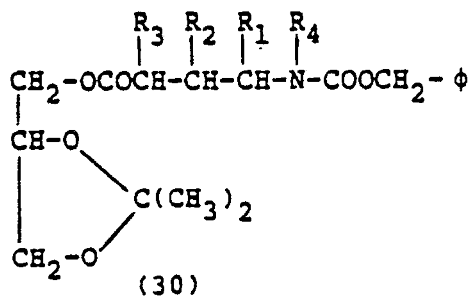

(30)

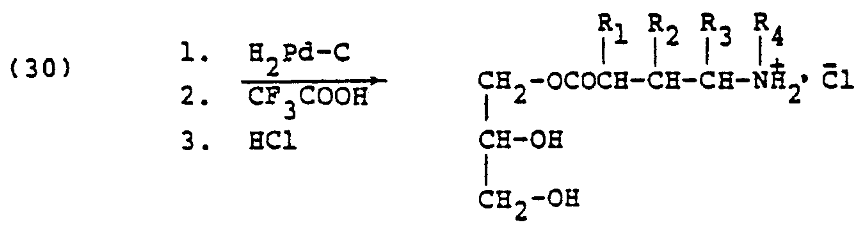


Glyceryl-GABA HCl
Glyceryl-GABA Analogue HCl
(31)

The various GABA and GABA analogue esters shown in Schemes II and III can also be synthesized on a microscale as radioactive derivatives using $^{14}C$-labeled t-BOC-GABA anhydride or t-BOC-GABA analogue anhydride with all the radioactivity present in the GABA or GABA analogue moiety of the molecule.

Ester derivatives of GABA and GABA analogue, with compounds such as long chain alcohols, cholesterol, and the like, and dexamethazone-like radicals, can be synthesized according to the reactions in Scheme IV.

SCHEME IV

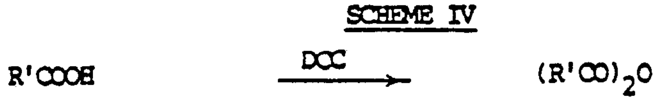


t-BOC-GABA or
t-BOC-GABA Analogue (2)

SYMM t-BOC-GABA-Anhydride
or
SYMM t-BOC-GABA-Analogue-Anhydride (3)

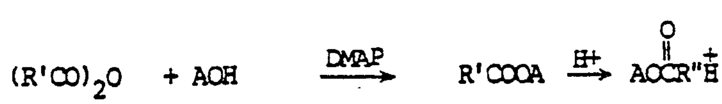


(4)

| A | COMPOUND |
|---|---|
| $-CH_2(CH_2)_7(CH=CH-CH_2)_3CH_3$ | Linolenyl-GABA or Linolenyl-GABA Analogue (5) |
| 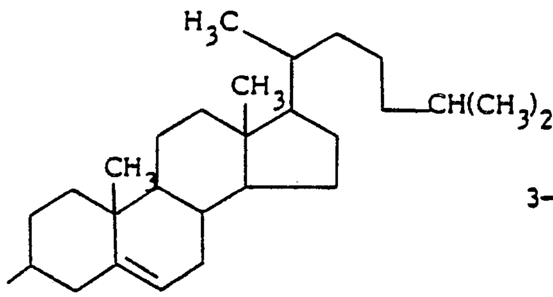 | 3-Cholesteryl-GABA or 3-Cholesteryl-GABA Analogue (6) |
| 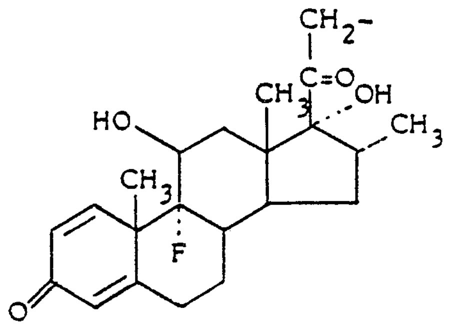 | Dexamethasone-21-GABA or Dexamethasone-21-GABA Analogue (7) |

DCC = dicyclohexyl carbodiimide
DMAP = 4-diemthylaminopyridine

The amino group of GABA or GABA analogue can be first protected, e.g., by formation of its t-butoxycarbonyl derivative (t-BOC—GABA or t-BOC—GABA analogue), by reaction with t-BOC—ON reagent. This product can be converted to the symmetrical anhydride by using, e.g., dicyclohexylcarbodiimide (DCC), Different alcohols can then be esterified by reaction with the GABA or GABA analogue anhydride to yield the GABA or GABA analogue ester products. After treatment of these compounds with a strong acid, e.g., trifluoroacetic acid, to remove the t-BOC group, the desired GABA or GABA analogue esters are obtained.

Similar methods on a microscale can be used to prepare these esters as radioactive derivatives with all the radioactivity present as $^{14}C$ in the GABA or GABA analogue moiety of the molecules. The IR and NMR spectra and elemental analysis of the compounds can be used to check consistency with their proposed structures.

Additional steps, such as those leading to further purification of the compounds, are also possible.

When the compounds of the invention are used to promote the uptake of GABA or GABA analogue by the brain, they are administered to an animal, e.g., a human, in need of higher brain levels of GABA, in an amount sufficient to promote the crossing of the blood-brain barrier of said animal by the compound.

When the ester derivatives of GABA or GABA analogue of the present invention are used for treating a condition associated with abnormal GABA in the brain, they are administered to an animal, e.g., a human, susceptible to said conditions in an amount sufficient to normalize said GABA levels.

When the ester derivatives of the present invention are used for treating a condition such as epilepsy, Huntington's disease, manic depression, general depression, schizophrenia and neuropsychiatric disorders, they are administered to an animal, e.g., a human, in need of such a treatment, in an amount sufficient to treat said condition.

When the present compounds are used to regulate general locomotor activity, they are administered to an animal, e.g., a human, in need of such regulation in an amount sufficient to regulate such activity.

When the ester derivatives of this invention are used for preventing or treating a condition associated with seizures, they are administered to an animal, e.g., a human, susceptible to seizures, in an amount sufficient to prevent or treat said seizures.

Administration may be by any method which allows the active compound to reach the bloodstream and penetrate through the blood-brain barrier. Typical methods include oral, rectal, peritoneal and topical administration of the compounds. When the compounds are administered orally, the composition can be in the form of dragees, tablets, syrups or ampules. When the compounds are administered rectally, the composition can be in the form of a suppository. In addition, when the compounds of the invention are to be administered by topical application, they can be in the form of a pomade or a gel.

The compounds of the invention can be prepared in pharmaceutical preparations containing the compounds themselves and a pharmaceutically acceptable carrier. The pharmaceutically acceptable carrier may be solid or liquid. Examples of liquid carriers include water, an aqueous solution of non-toxic salts, such as sterile physiological solutions of saline, or aqueous solutions containing organic solvents, such as ethanol. Also suitable are emulsions, such as oil-in-water emulsions. Solid carriers include both nutritive carriers, such as sucrose or gelatin, and non-nutritive carriers, such as cellulose, alphacyclodextrin, or talc. Slow-release capsules and other protective means are particularly suitable for the oral administration of the present compounds due to the protection afforded against hydrolysis in the gastrointestinal tract. Preferred are those capsules which permit the compounds to bypass the stomach. When the present compounds are to be administered peritoneally, they can be administered by subcutaneous, intramuscular or intravenous injections.

Amounts of ester derivatives of GABA or GABA analogue useful for promoting the uptake of GABA or GABA analogue by the brain may vary from individual to individual, and can be determined by experimentation as is well understood by those skilled in the pharmaceutical arts. For intravenous injection, amounts in the range of about 1—1000 μmol/kg body weight are preferred. Also a preferred range is between about 1—500 μmol/kg. Especially preferred are amounts in the range of 1—100 μmol/kg body weight. Compounds of the invention have been found to be effective on mammals (mice) within this range.

Generally, compounds are more active when administered intravenously than by the other preferred routes. For oral, topical, or rectal administration, as well as for the subcutaneous, or intramuscular injection of the compounds, amounts in the range of about 1—2000 μmol/kg body weight, are preferred. Also a preferred range is between about 1—1000 μmol/kg body weight. Especially preferred are amounts in the range of about 1—200 μmol/kg.

The above disclosure generally describes the present invention. A more complete understanding can be obtained by reference to the following specific examples which are provided herein for purposes of illustration only and are not intended to be limiting unless otherwise specified.

## EXAMPLES

### Synthesis of Ester Derivatives of GABA

Melting points were determined on a hot stage apparatus and are uncorrected. IR spectra were obtained in a Perkin-Elmer Infracord spectrophotometer and are reported in $cm^{-1}$; NMR spectra were determined in a CFT 20 using tetramethylsilane as an internal reference. Elemental analyses for C, H and N were carried out by Midwest Micro Labs Ltd., IN; all compounds analyzed within ±0.4% of their theoretical values. Thin-layer chromatographic (TLC) determinations were carried out on silica gel-coated plastic TLC sheets from Eastman Kodak using the following solvent systems (in vols):

(A) chloroform, acetic acid (9:1);
(B) chloroform, methanol (9:3);
(C) propanol, acetic acid, water (4:1:1);
(D) chloroform, methanol, acetic acid (90:30:3);
(E) chloroform, methanol, acetic acid (18:6:1);
(F) ethyl acetate, acetic acid, ethanol (9:1:1);
(G) benzene, acetone (9:1);
(H) ethylacetate, hexane (1:1).

### Example 1
### Synthesis of the Butyl Ester of GABA

The butyl ester of GABA was prepared in very high yield by direct esterification of GABA with n-butanol by the method shown in Scheme I in the specification.

A solution of 100 mg of γ-aminobutyric acid in 30 ml anhydrous n-butanol (dried over calcium hydride and distilled) was saturated with anhydrous HCl gas and sealed in a glass ampule. The mixture was heated over a steam bath for 16 hr and then evaporated to dryness to yield a white solid which was crystallized from an ethanol/ether mixture. The yield obtained was 92% based on GABA. The compound had the following characteristics:

Melting point: 72°C
TLC in solvent A: $R_F$ 0.44 (single spot)
IR (Neat): 3550, 1730

The radioactive n-butyl ester of GABA was prepared as above using 4 mg of GABA, 50 μCi of (U, $^{14}C$)GABA (New England Nuclear, Boston, MA) and 3 ml of anhydrous butanol as starting materials. The radioactive product was obtained in 82% yield with a specific activity of 186 μCi/mM. On TLC plates all of the radioactivity (99%) was found to be associated with a single spot which co-migrated with the unlabeled ester at $R_F$ 0.44 in solvent A. After hydrolysis of the ester with alkali, all the radioactivity was transferred to the position of GABA on TLC plates. The yield for this reaction, and the activity of the $^3H$ butyl ester of GABA are shown in Table I, hereinafter.

Example 2

Synthesis of the 9,12,15-Octadecatrienyl Alcohol (Linolenoyl), Cholesteryl, and Dexamethasone Esters of GABA

These ester derivatives of GABA were synthesized by esterification of the corresponding alcohol with the symmetrical anhydride of t-butoxycarbonyl-GABA according to Scheme IV in the specification.

The t-butoxycarbonyl-aminobutyric acid (t-BOC-GABA, Compound 2)

This compound was prepared by the reaction of GABA with a t-BOC derivative (2-(t-butoxycarbonyl oxiimino)-2-phenylacetonitrile). The BOC-ON compound was obtained in 96% yield according to published methods. The product had a melting point of 52—54° in agreement with literature values and migrated as a single spot on TLC plates. $R_F$ values were 0.84 and 0.71 in solvents E and F, respectively. ($^{14}C$)t-BOC-GABA was also prepared from 30 mg quantities GABA and 100 μCi uniformly labeled $^{14}C$-GABA (New England Nuclear).

Symmetrical t-BOC-GABA Anhydride (Compound 3):

A cold solution (0°C) of 558 mg dicyclohexyl carbodiimide in 8 ml of dry methylene chloride was added to a cold solution of t-BOC-GABA 1.05 g in 7 ml of dry methylene chloride. An immediate precipitate of dicyclohexylurea was formed. The mixture was then stirred for an additional 2 hr at room temperature and then filtered to remove the urea derivative. The filtrate was evaporated on a rotary evaporator. The solid product obtained was redissolved in 20 ml of anhydrous ethyl acetate and kept overnight at 4°C to allow further precipitation of any remaining dicyclohexylurea. The resulting solution was then filtered, concentrated to 8 ml *in vacuo*, and dry petroleum ether was added to precipitate a white compound. This compound was isolated by filtration, washed with a mixture of ethyl acetate/petroleum ether (1/4, vols), dried *in vacuo* over $P_2O_5$ and then crystallized from the solvent mixture to yield 915 mg of the anhydride (87%). The compound had the following characteristics:

Melting point: 160°C,
TLC in solvent F: $R_F$ 0.74
IR (Nujol): 3480, 3400, 1810, 1730, 1680
Anal. (C,H,N): $C_{18}H_{32}N_2O_7$

The radioactive anhydride was synthesized as above with an 84% yield, using 15 mg of $^{14}C$-labeled t-BOC-GABA as a starting material. The product had a specific activity of 2 mCi/mM and an $R_F$ value identical to the unlabeled compound.

t-BOC-GABA Cholesterol Ester (Compound 8):

A solution of 1.63 g of t-BOC-GABA anhydride in 25 ml of tetrahydrofuran (distilled just before use from calcium hydride) was added to a solution of 1.35 g of cholesterol and dry tetrahydrofuran, followed by 427 mg of dry 4-dimethylaminopyridine. The mixture was stirred under nitrogen for 48 hr at room temperature and then evaporated to dryness to give a waxy solid. The product was dissolved in 7 ml of ethyl acetate, washed (3×) with 15 ml of 5% (w/v) sodium bicarbonate and then (3×) with 15 ml of distilled water. The ethyl acetate solution was separated and dried over anhydrous sodium sulfate, filtered and then evaporated to dryness. The product was recrystallized from acetone/water to yield 1.95 g of the t-BOC-GABA cholesterol ester having the following characteristics:

Melting point: 122.5°C
TLC: $R_F$ 0.98 and 0.52 in solvents D and G, respectively

The $^{14}C$-labeled compound was synthesized as above using 300 mg of cholesterol as a starting material.

Yield: 83%
Specific activity: 13.6 μCi/mM
IR: 3450, 1730, 1690, 1700
NMR ($CDCl_3$): δ 5.35 (bm, 1H), 4.55 (m, 2H) 3.15 (m, 2H), 2.10—2.40 (m, 4H); 1.45 (s, 9H)
Anal: $C_{36}H_{61}NO_4$ (C, H, N).

(3-Cholesteryl)-gamma-Aminobutyrate (Compound 6):

A solution of γ-t-BOC amino-butyrylester of cholesterol (100 mg, 175 μmol) in 0.53 ml of 4 M HCl in dioxane and 2.0 ml of dry dioxane was stirred at room temperature for 70 min. The solution was concentrated *in vacuo* at room temperature, and the white solid thus obtained was washed repeatedly with ethyl-acetate and dried under vacuum over $P_2O_5$ to give 50 mg of the 3-cholesteryl-GABA ester Compound *6* (Yield 60%). The compound had the following characteristics:

TLC in solvent E $R_F = 0.38$ and
in solvent B, $R_F = 0.33$

Since the amine hydrochloride was very hydroscopic, good IR and NMR spectra could be obtained only by making the N-acetyl derivative by acetylation with acetic anhydride in pyridine. The N-acetyl derivative had the following characteristics:

IR (Neat): 3350, 1720, 1630
NMR in ($CDCl_3$): δ 5.3 (bm, 1H), 3.2 (m, 2H), 2.3—1.0 (bm), 1.96 (s, 3H), 1.26 (s, 3H), 0.9 (s, 3H)
Anal.: $C_{32}H_{54}NO_2$ Cl·$H_2O$ (C, H, N)

9,12,15-Octadecatrienyl-4-(t-butoxycarbonyl)aminobutyrate (linolenoyl ester of t-BOC-GABA) (Compound 9):

To a solution of linolenyl alcohol (285 mg, 1.05 mmol) in 35 ml of benzene under an atmosphere of nitrogen was added t-BOC-GABA anhydride (480 mg, 1.24 mmol) and 4-dimethylaminopyridine (150 mg, 1.23 mmol). The mixture was stirred at room temperature for 4 hr. The solution was then washed with 5% sodium bicarbonate, ice-cold 0.1N HCl solution and finally with ice-cold water. The organic layer was then dried over anhydrous sodium sulfate and concentrated to yield 370 mg (76%) of the ester (Compound *9*). The compound had the following characteristics:

TLC in solvent H: $R_F = 0.74$

IR (Neat): 3500, 1720, 1160

NMR in ($CDCl_3$): 5.36 δ (m, 6H), 4.55 (bm, 1H), 4.06 (t (J=6.5Hz), 2H), 3.15 (m, 2H), 2.8 (m, 4H), 2.29 (m, 2H), 2.0—1.3 (bm, 18H), 1.43 (s, 9H), 0.97 (t (J=7.5Hz), 3H)

A similar procedure was used to prepare the $^{14}C$-labeled derivative using ($^{14}C$)t-BOC-GABA anhydride and unlabeled cholesterol (10 mg) with a yield of 70%.

TLC in Solvent H: $R_F = 0.73$

Linolenoyl Ester of γ-aminobutyric Acid (Compound 5):

A solution of t-BOC-GABA ester of linolenyl alcohol (330 mg, 0.74 mmol) in 20 ml of methylene chloride was cooled with ice under nitrogen atmosphere. Trifluoroacetic acid (0.5 ml) was added to the solution, and the mixture was stirred at 0°C for 1 hr, and at room temperature for an additional 2 hr. The solution was concentrated in aspirator vacuum, and the residue was taken up in 20 ml of chloroform and washed with 5% (w/v) sodium bicarbonate solution and with water. The chloroform layer was dried over anhydrous magnesium sulfate and was concentrated under vacuum to yield a light yellow liquid, 200 mg (0.58 mmol) (yield 78%). Because of its hygroscopic properties, the linolenoyl ester of GABA (Compound *5*), was converted to its N-acetyl derivative with acetic anhydride in pyridine prior to its characterization and elemental analysis. The characteristics of the compound were as follows:

TLC in solvent B: $R_F = 0.43$

IR (Neat): 3300, 1730, 1650, 1550

NMR ($CDCl_3$): 5.36 (m, 6H), 4.05 (t (J=4.8Hz), 2H), 32.8 (m, 2H), 2.8 (m, 4H), 2.35 (t (J=6.6Hz), 2H), 2.0—1.30 (bm, 18H), 1.95 (s, 3H), 1.59 (s, 3H), 0.97 (t (J=7.5Hz), 3H)

Anal.: ($C_{24}H_{41}NO_3$) C,H,N.

The radioactive derivative was prepared from $^{14}C$-labeled t-BOC-GABA linolenoyl ester (7 mg) as above. The specific activity of the final compound was 120 μCi/mol.

9-Fluoro-11β,17-dihydroxy-21-(N-gamma-t-BOC aminobutyroxy)-16α-methyl pregna-1,4-diene-3,20-dione(21-(N-gamma-t-BOC-aminobutyroyl)dexamethasone) (Compound 11):

A solution of dexamethasone (Sigma) (120 mg, 0.31 mmol), t-BOC-GABA anhydride (144 mg, 0.37 mmol) and 4-dimethylaminopyridine (45 mg, 0.37 mmol) in 20 ml of dry methylene chloride was stirred at room temperature for 70 min. The solution was washed serially with 5% (w/v) sodium bicarbonate solution, 0.1N HCl and brine. The organic layer was dried over anhydrous $Na_2SO_4$ and was concentrated *in vacuo* to obtain 0.164 g (0.28 mmol) of the ester (Compound 11) with a yield of 92%. The characteristics of the ethyl acetate derivative were as follows:

M.P. 117—9°C

TLC $R_F = 0.74$ (ethyl acetate)

$R_F = 0.38$ (dexamethasone)

IR ($CHCl_3$): 3500, 1710, 1660.

NMR ($CDCl_3$): δ 7.27 (d (J=10.5Hz) 1H) 6.37—6.1 (m, 2H), 4.89 (s, 2H) 4.75—4.05 (m, 2H), 3.19 (m, 2H) 2.48 (m, 2H) 1.63 (s, 3H), 1.54 (s, 2H) 1.44 (s, 9H), 1.05 (s, 3H) 0.91 (d (J=7.3Hz), 3H)

Anal: ($C_{31}H_{44}FNO_8$) C,H,N

The radioactive derivative was prepared as above using $^{14}C$-labeled t-BOC-GABA anhydride and 10 mg of dexamethasone.

Dexamethasone-21-gamma-aminobutyrate (Compound 7):

A solution of dexamethasone-21-N-gamma-t-BOC aminobutyrate (400 mg, 0.69 mmol) and trifluoroacetic acid, 0.2 ml in 20 ml of methylene chloride was stirred at room temperature for 1 hr, and the mixture was concentrated *in vacuo*. The residue was taken up in 9 ml of 0.1N HCl and was lyophilized. The crude product was purified on a silica gel column (20 × 1 cm) eluting with $CHCl_3$, $CH_3OH$ (40:10 vols). The eluate was evaporated to dryness and the residue was taken up in 10 ml water and was freeze-dried to obtain the dexamethasone GABA ester product (Compound *7*) as the trifluoroacetate monohydrate, a fluffy solid, 207 mg (yield 58%). The characteristics of the compound were as follows:

TLC in solvent B: $R_F = 0.76$

in solvent C: $R_F = 0.52$

M.P.: 185—88°C

IR (Neat): 3500, 1720, 1670, 1650, 1620

NMR (acetone $D_6$): δ 7.26 (m, 1H), 6.0—6.24 m, 2H), 4.99 (d (J=1.9Hz), 2H), 4.1—4.7 (bm, 2H), 3.84 (m, 2H) 3.30 (s, 3H), 2.40—2.86 (m, 14H) 1.61 (s, 3H), 0.98 (m, 3H)

Anal: (trifluoroacetate) ($C_{28}H_{37}F_4NO_8 \cdot H_2O$) C,H,N.

The $^{14}C$-labeled dexamethasone-12-γ-aminobutyrate was obtained from the t-BOC derivatives as above using 6 mg of starting material. The characteristics of the radiolabeled compound were as follows:

Yield: 75%

Specific activity: 34 μCi/mmol.

The yields for the overall synthesis of some of these compounds labeled in the GABA radical are shown in Table I, hereinafter. The results of the elemental analysis of compounds 2, 4, 5, 6, 9, 11 and 12 are shown in Table II also hereinafter.

Table I. SYNTHESIS OF ESTER DERIVATIVES OF GABA

| GABA DERIVATIVE | YIELD | SPECIFIC ACTIVITY[a] μCi/mM |
|---|---|---|
| glyceryl-GABA | 40% | 645 |
| dexamethasone-GABA | 28% | 34 |
| inositol-mono GABA | 82% | 4.2 |
| 3-glucosyl-GABA | 87% | 228 |
| butyl-GABA | 92% | 3680[b] |
| linolenoyl-GABA | 70% | 120 |
| glyceride linolenoyl diGABA | 75% | 139 |
| di linolenoyl monoGABA-glyceride | 60% | 41 |
| inositol-diGABA | 35% | 7.1 |
| cholesteryl-GABA | 86% | 13.6 |

a - $^{14}C$ labeled in the GABA radical of molecule

b - $^{3}H$ labeled in the GABA radical of the molecule

TABLE II. ELEMENTAL ANALYSIS RESULTS

| | THEORETICAL | | | OBSERVED | | |
|---|---|---|---|---|---|---|
| COMPOUND | %C | %H | %N | %C | %H | %N |
| SCHEME IV | | | | | | |
| 3 | 55.65 | 8.30 | 7.21 | 55.61 | 8.37 | 7.18 |
| 6 | 70.78 | 10.65 | 2.66 | 70.72 | 10.28 | 2.82 |
| 7 | 55.17 | 6.40 | 2.30 | 55.51 | 5.94 | 2.26 |
| 8 | 75.61 | 10.75 | 2.45 | 75.75 | 10.83 | 2.42 |
| 10 | 73.60 | 10.46 | 3.58 | 73.15 | 10.34 | 3.59 |
| 11 | 64.42 | 7.62 | 2.42 | 64.03 | 7.68 | 2.37 |
| SCHEMES II AND III | | | | | | |
| 2 | 73.47 | 10.20 | | 73.29 | 10.37 | |
| 4 | 67.04 | 9.50 | 2.61 | 66.61 | 9.77 | 2.83 |
| 5 | 64.82 | 9.14 | 3.88 | 64.56 | 9.34 | 4.06 |
| 6 | 72.22 | 9.98 | 1.76 | 71.69 | 10.37 | 1.75 |
| 9 | 61.64 | 7.12 | 3.99 | 61.30 | 7.29 | 3.82 |
| 11 | 65.35 | 8.91 | 4.62 | 65.60 | 9.24 | 4.46 |
| 12* | 71.33 | 9.91 | 1.85 | 71.18 | 10.47 | 1.91 |

* Was hygroscopic and highly unstable, and the values represent one mole of water of hydration.

Biological Studies with the Compounds of Examples 1 and 2

Comparative Brain/Liver Uptake of GABA

The uptake of a $^{14}C$-labeled compound into brain was compared with the uptake into liver of the same animal. Table III summarizes the results of the application of this method to $^{14}C$-labeled GABA. The data indicate that the BPI values (mean ± S.E.M. = 0.96 ± 0.09%) were independent of the route of administration (i.p., s.c., or i.v.) of the compound and the dose (30—380 μmol/kg) used.

TABLE III. UPTAKE OF ($^{14}$C)GABA BY MOUSE TISSUES

| DOSE (μmol/kg) | INJECTION ROUTE | (BRAIN) (nmol/g) | (LIVER) (nmol/g) | BPI (5 min) (%) |
|---|---|---|---|---|
| 30 | i.v. | 0.04 | 4.2 | 0.95 |
| 60 | i.p. | 0.23 | 29 | 0.80 |
| 120 | i.p. | 0.45 | 46 | 1.00 |
| 160 | i.p. | 0.66 | 71 | 0.93 |
| 210 | i.p. | 0.83 | 83 | 1.00 |
| 270 | s.c. | 1.03 | 104 | 1.00 |
| 380 | s.c. | 1.81 | 165 | 1.10 |

Mean ± S.E.M.: 0.96 ± 0.09
BPI: (Brain/Liver) x 100

Comparative Brain/Liver Uptake of the GABA Esters

Table IV gives the results obtained for the brain uptake of the GABA esters.

TABLE IV - TIME COURSE OF UPTAKE OF ($^{14}C$) LABELED GABA ESTERS

| GABA ESTER | DOSE (μmol/kg) | TIME (Min) | (BRAIN) (nmol/g) | (LIVER) (nmol/g) | BPI (%) |
|---|---|---|---|---|---|
| n-butanol-GABA *(1)* | 380 | 5 | 19.2 ± 0.3 | 26.0 ± 1.6 | 74 ± 3.4 |
| | 380 | 60 | 14.4 | 13.7 | 105 |
| 1-Linolenoyl-GABA *(5)* | 260 | 5 | 3.5 ± 0.4 | 141.7 ± 8 | 2 ± 0.6 |
| | 260 | 15 | 11.0 | 220.8 | 5 |
| | 260 | 45 | 24.9 | 33.3 | 75 |
| Cholesteryl-GABA *(6)* | 50 | 5 | 0.17 ± 0.03 | 0.69 ± .08 | 25 ± 7 |
| | 50 | 15 | 0.67 | 1.19 | 56 |
| | 50 | 60 | 0.79 | 0.92 | 86 |
| | 50 | 180 | 0.60 | 1.89 | 32 |
| Dexamethasone GABA *(7)* | 70 | 5 | 8.7 ± 1 | 10.8 ± 2 | 81 ± 20 |
| | 70 | 15 | 21.5 | 29.0 | 74 |

BPI = Brain Penetration Index = (Brain)/(Liver) x 100. ($^{14}C$)labeled compounds were injected s.c. into mice in 0.5 ml of 25% (vols) propylene glycol in water. The data for the 5-min BPI measurements are mean ± S.E.M. (n = 3 animals).

The 5-min PBI data indicate that the uptake of the dexamethasone, n-butyl, cholesteryl and 1-linolenoyl esters are, respectively, increased by a factor of 81-, 74-, 25- and 2-fold over GABA. Apparently, the enhanced lipid solubility of the molecules facilitated their uptake into the brain. Results at longer times than 5 min indicate that accumulation of radioactivity in brain persisted for 45—60 min post-injection for the linolenoyl and cholesteryl esters, whereas the concentrations in the liver were more variable over time. These results indicate that the GABA esters can enter the CNS and may remain there for at least 1 hr or 2 hr. TLC analysis of brain homogenates at 1 hr after administration indicates that about 70% of the labeled cholesteryl GABA and 4% of the butyl GABA ester are present as intact molecules. The remaining radioactivity migrated in the GABA region of the chromatogram.

All determinations were carried out at 5 min after injection. The volumes of the injected doses into mice (body weight, 18—24 g) for the i.v., i.p., and s.c. doses were 0.15, 0.3 and 0.5 ml, respectively. The measured concentrations in brain and liver represent the actual amounts of GABA found by thin-layer chromatographic (TLC) analysis of the tissue homogenates. Each tissue was homogenized in 8 ml of pyridine in a glass homogenizer. The precipitated material was removed by centrifugation for 10 min at 12,000 × *g* and the supernatant was evaporated to dryness under $N_2$ and analyzed by TLC in Solvent A. This procedure insured that only labeled GABA, and not its metabolites, was being measured. The mean BPI for labeled GABA was 0.96 ± 0.09% (n = 7) and was apparently not dependent on the route of injection and the dose (between 30 and 380 μmol/kg).

Each labeled test compound was injected as a solution in 0.5 ml of saline (Compound *7*) or in 25% propylene glycol in water (Compounds *1, 5, 6*) subcutaneously (s.c.) into young adult Balb/c mice (18—24 g body weight). After 5 min (or longer) animals were sacrificed and the brain and liver removed, weighed and homogenized respectively in 8 ml and 10 ml of brain protein solvent (1% (w/v) sodium dodecyl sulfate in 6 M urea and 19 mM EDTA, pH 7.4, 0.03 M phosphate). Aliquots (0.5 ml) of the homogenates were mixed

with 10 ml of Aquasol 2 (New England Nuclear) and counted in a Beckman liquid scintillation counter. The total uptake of each compound per g brain and liver tissue was calculated. The proportion of uptake into brain as compared to liver (100%) of the same animal was designated as the Brain Penetration Index (BPI), expressed as a percentage typically at 5 min after injection.

Pharmacological Studies with the Compounds of Examples 1 and 2

Two test methods were used to evaluate the neuropharmacological activities of the GABA esters.

(1) General locomotor activity of mice

The effect of intraperitoneal (i.p.) injections of test compounds on the general motor activity of mice during a 60-min period was determined in a Stoelting electronic activity monitor. Dose-response curves were obtained from which the half-maximally effective values were calculated and the results are shown in Table V.

TABLE V - PHARMACOLOGICAL TESTING OF GABA ESTERS GENERAL MOTOR ACTIVITY DATA

| COMPOUND | DOSE (μmol/kg, i.p.) | GENERAL MOTOR ACTIVITY |
|---|---|---|
| GABA | 1942 | 100±15 |
| n-Butyl-GABA | 307 | 89± 9 |
| | 654 | 83±15 |
| 1-Linolenoyl-GABA | 34 | 90± 2 |
| | 103 | 74± 3* |
| Cholesteryl-GABA | 6 | 67± 3* |
| | 21 | 49± 4** |
| | 65 | 15±14** |
| Dexamethasone-GABA | 27 | 94±10 |

Cumulative activity scores over 60 min post-injection of drug expressed as mean percentage of appropriate vehicle control for each drug. (x ± S.E.M., n = 6)
(*) $p < 0.01$
(**) $p < 0.001$ by t-test

GABA itself had no significant effect at a dose of 1.94 mmol/kg, i.p., or when 500 μg of the compound was administered intracisternally directly into the cerebrospinal fluid. This observation is similar to previous experiments in which systemically injected GABA doses of up to 1 g/kg (9.7 mmol/kg) had little effect on motor activity in rats.

The dexamethasone, butyl and linolenyl esters were not active by this behavioral test, but the cholesteryl ester was active in reducing the general activity of test mice as shown in Table V.

Dose-response measurements in rats and mice are shown in Fig. 1. The data indicate that motor activity was reduced by 50% at i.p. injected doses of 8 and 17 μmol/kg, respectively, in the two species. The amount of the compound present in the mouse brain at 5 min after a dose of 50 μmol/kg, considerably large than the $ED_{50}$ of 17 μmol/kg, was only 0.17 nmol/g (Table IV) and so indicates that cholesteryl-GABA is a highly active compound in mice. This result is comparable to the effects of direct administration of muscimol (1.75 nmol/g) and baclofen (0.25 nmol/g) by cannula into the nucleus accumbens of rat brain.

The compounds were dissolved in 25% (vols.) propylene glycol in water and injected i.p. into Balb/c mice (18—24 g). Dose-response measurements were obtained for 6 animals per dose or time point. Activity was monitored for 60 min using a Stoelting Electronic Activity Monitor (EAM) apparatus to assess the

general motor activity of the animals. The dose at which a compound reduced such spontaneous motor activity by 50% in comparison to the control (vehicle-injected and uninjected) animals was used as a measure of pharmacological activity.

Anticonvulsant Activity in Mice

In a second behavioral test, the ability of a substance to prevent or delay the onset of biculline-induced epileptic seizures was measured. There were no significant effects at 1 or 6 hr. At 24 hr after i.p. injection of the cholesteryl derivative at a dose of 30 mg/kg (64 μmol/kg), the onset of seizures was delayed by 30% after an acute i.p. injection of bicuculline at 0.3 mg/kg. In contrast, the linolenyl ester of GABA had the surprising property of accelerating the seizure onset time by 70% at a dose of 50 mg/kg, even though it had an insignificant effect on the general motor activity of mice (Table V). The dexamethasone and butyl esters had no effect.

Since these changes were detected at 24 hr after administration of the GABA esters, it is not known whether they are due to the properties of the intact ester itself, to free GABA, or to other metabolites. Nevertheless, these results suggest that the cholesteryl ester of GABA had a central depressant effect but had only a slight anticonvulsant property in the test used.

The compounds were also injected i.p. at 1, 6 and 24 hr before the administration of bicuculline (0.3 mg/kg) s.c. The latency to the onset of generalized tonic-clonic epileptic seizures and the protection against the lethality of bicuculline were measured and evaluated as a percent of the data for vehicle-injected controls. No significant changes were obtained at the 1 hr and 6 hr time points. At the 24 hr time, however, some compounds delayed while others accelerated the onset of seizures.

Example 3

Synthesis of Glyceryl Esters of GABA Further Substituted by Linolenoyl Radicals

Two of the hydroxyl groups of glycerol were first protected by forming a glycerol acetonide:

1-Linolenoyl-2-,3-O-isopropylidine glycerol

a) *Using Linolenoic anhydride:*

A solution of glycerol acetonide 470 mg, 3.55 μmol), linolenic anhydride (from Sigma Chemical Co., St. Louis) (1.902 g, 3.5 mmol) and 4-dimethylaminopyridine (460 mg, 3.8 μmol) in dry benzene was stirred at room temperature under $N_2$ atmosphere for 8 hr. The benzene solution was washed with 5% (w/v) sodium bicarbonate solution (2 × 20 ml), 0.1N HCl solution (2 × 20 ml) and with brine (2 × 20 ml). The organic layer was dried over anhydrous magnesium sulfate and concentrated under vacuum to get a crude yield of 2.178 g. This material was purified by passage through a silica gel column by eluting with 5% ethyl acetate in petroleum ether to give 953 mg (2.4 μmol) of the pure compound (yield 69.5%). The characteristics of the thus obtained compound were as follows:

TLC: $R_F$ 0.66 ethyl acetate:pet ether (1:1)

IR (Neat): 1730 (C=0), 1370

NMR ($DCDl_3$): δ 5.35 (m, 6H, vinyl-H) 3.6—4.35 (m, 5H, glycerol $CH_2$, CH) 2.75, (m, 4H), 2.25 (1, J=8Hz, 2H), 1.70—2.15 (m, 4H), 1.15—1.70 (bs, 16H), 0.95 (t, J=8Hz, 3H)

Anal.: ($C_{24}H_{40}O_4$) C,H

b) *Using Linolenoyl chloride:*

To a solution of 660 mg (5 mmol) of glycerol acetonide (20) and 620 mg (5 mmol) of 4-dimethylaminopyridine (DMAP) in 30 ml of dry benzene under $N_2$ atmosphere was added a solution of linolenoyl chloride (Eastman, freshly distilled) 1.49 g (5 mmol) in 20 ml benzene from a pressure equalizing funnel over 45 min. The DMAP hydrochloride was precipitated as a white solid during the reaction. The reaction mixture was treated as above, after stirring for another 2.5 hr. The crude product (2 g) was distilled to get 1.32 g of Compound *21* (yield 67%) (b.p. 155—60°C/0.05 min).

Symmetrical t-BOC-GABA anhydride:

The compound was prepared by reacting t-BOC-GABA or t-BOC ($^{14}$C)GABA with dicyclohexyl carbodiimide.

1-Glyceryl linolenoate (Compound 22):

A solution of 1.2 g (3.1 μmol) of the linolenoyl ester of glycerol acetonide in 60 ml of dry methylene chloride was stirred with 2.2 ml of trifluoracetic acid at 0°C for 12 h under $N_2$. The solution was concentrated in a rotary evaporator, and traces of the trifluoracetic acid were removed under high vacuum. The crude material was purified by chromatography on a silica gel column, eluting with ethyl acetate (yield 30%) in petroleum ether to obtain 730 mg (2.1 μmol) (yield 68%). The product (Compound *22*) migrated as a single spot on TLC plates and had NMR and IR spectra consistent with their proposed structure. This was used without further purification for the next step. The characteristics of this compound were:

TLC: $R_f$ 0.12 ethyl acetate:pet ether,

IR (Neat) 3500—3700, 1725

NMR ($CDCl_3$): δ 5.25—5.46 (m, 6H, *CH*=*CH*) 3.60—4.20 (m, 5H, glycerol $CH,CH_2$) 2.68—2.80 (m, 4H), 1.88—2.45 (m, 6H) 1.31 (bs, 12H), 0.97 (t, J=9Hz, 3H).

1-linolenoyl-3-(N-gamma-t-BOC aminobutyroyl)glycerol (Compound 23):

t-BOC-GABA anhydride (250 mg, 640 µmol) and 4-dimethlyaminopyridine (80 mg, 660 µmol) were added to a solution of 1-linolenoyl glycerol (470 mg, 1.34 mmol) in 50 ml of dry benzene. The mixture was stirred overnight under $N_2$ atmosphere. The benzene solution was then extracted with 0.1N HCl solution, 5% (w/v) aqueous $NaHCO_3$ and water. The organic layer was removed and dried over anhydrous $MgSO_4$ and concentrated *in vacuo* to yield 504 mg of crude product. This material (Compound *23*) was then purified by chromatography in a silica gel column, eluting with 30% ethyl acetate in petroleum ether to yield 234 mg (440 µmol) of a compound (TLC pure) as a viscous liquid (yield 68%). The characteristics of the compound were found to be as follows:

TLC: $R_f$ 0.45 ethyl acetate:pet ether, 1:1

IR (Neat) 3600, 1725, 1680

NMR ($CDCl_3$): δ 5.29—5.43 (m, 6H, vinyl-H) 4.15 (m, 5H glycerol CH,$CH_2$) 3.13—3.21 (m, 2H), 2.80 (m, 4H) 2.31—2.39 (m, 4H, 1.45—2.25 (m, 6H) 1.43 (s, 9H), 1.25 (bs, 12H) 0.97 (t (J-9Hz), 3H)

Anal: ($C_{30}H_{51}NO_7$) C,H,N.

The same procedures were used in a microscale (20 mg) to synthesize the compound as a radioactive derivative using $^{14}C$-labeled t-BOC-GABA anhydride.

1-linolenoyl-2,3-di(N-gamma-t-BOC-aminobutyroyl)propane-1,2,3-triol (Compound 24)

The symmetrical anhydride of t-BOC GABA (567 mg, 1.45 mmol) and 4-dimethlyaminopyridine, 178 mg (1.45 mmol) were added to a solution of 1-linolenoyl glyceride (256 mg, 730 µmol) in 50 ml of dry benzene. The mixture was stirred for 6 hr under nitrogen atmosphere. The reaction product as a solution in benzene was extracted with the following, in sequence: 0.1 N HCl (2 × 20 ml), 5% (w/v) $NaHCO_3$ solution (2 × 20 ml) and with brine (2 × 20 ml). The organic layer was separated and dried over anhydrous $MgSO_4$ and concentrated *in vacuo* to yield 1.23 g of the crude product (Compound *24*). The material was then passed through a silica gel column. Elution with petroleum ether:ethyl acetate (80:20) yielded the pure product, 356 mg, 0.49 mmol (yield 68%). The characteristics of the compound are as follows:

TLC: $R_f$ 0.67 in ethyl acetate:pet ether, 1:1

IR (Neat) 3500, 1725, 1695

NMR ($CDCl_3$): δ 5.35 (bm, 6H, *CH*=*CH*) 3.95—4.75 (m, 5H glycerol-C$H_2$,CH) 3.1 (m, 4H), 2.75 (m, 4H) 2.1—2.5 (bm, 6H), 1.5—2.1 (m, 8H) 1.45 (s, 18H), 1.3 (bs, 12H) 0.97 (t, J=8Hz, 3H)

Anal: ($C_{39}H_{66}N_2O_{10}$) C,H,N

1,2-dilinolenoyl-3-(N-gamma-t-BOC aminobutyroyl)propane-1,2,3-triol (Compound 25):

A solution of linolenoyl chloride (11 mg, 380 mol) in 10 ml of dry benzene was added dropwise from a pressure equalizing funnel to a solution of 1-linolenoyl-3-(N-gamma-t-BOC aminobutyroyl)glycerol (Compound *23*) (193 mg, 361 µmol) and 4-dimethylaminopyridine (55 mg, 0.47 mmol) in 15 ml of dry benzene at 0°C under $N_2$. The mixture was stirred for 4 hr at about 10°C, after which the benzene solution was washed with 0.1N HCl, 5% (w/v) $NaHCO_3$ and finally saturated NaCl solution. The organic layer was separated, dried over anhydrous $MgSO_4$, and concentrated *in vacuo* to yield 223.7 mg of crude material. This was adsorbed onto a silica gel column and eluted with 15% ethyl acetate in petroleum ether to yield 186.4 mg (yield 65%) of the purified Compound *25*.

TLC: $R_f$ 0.40 ethyl acetate:pet ether, 1:9

IR (Neat) 3450, 1730, 1710

NMR ($CDCl_3$): δ 5.36 (9H, m, vinly H) 4.21 (m, 5H glycerol CH,$CH_2$) 3.15 (m, 2H), 2.80 (m, 6H) 1.452.40 (m, 16H), 1.43 (s, 9H) 1.31—1.25 (bs, 20H), 0.97 (m, 6H)

Anal: ($C_{48}H_{70}NO_8$) C,H,N.

1-linolenoyl-2,3-(gamma-aminobutyroyl)propane-1,2,3-triol (Compound 26)

The 1-linolenoyl-2,3di(N-gamma-t-BOC aminobutyroyl ester) (Compound *24*) (200 mg, 277 µmol) in 50 ml of dry methylene chloride containing 2.2 ml of trifluoroacetic acid was stirred at 0°C under $N_2$ for 6 hr. The solution was concentrated under vacuum and was taken up in 15 ml of ethyl acetate. The ethyl acetate solution was treated for 2 min with saturated NaCl solution adjusted to pH 8.5 with $NaHCO_3$. The organic layer was separated, dried over anhydrous $Na_2SO_4$ and concentrated under vacuum to obtain 98 mg (188 mol) of the diamine (Compound *26*) (yield 68%). The other characteristics of the compound were as follows:

TLC $R_f$ 0.77 in BuOH:AcOH:pyridine:water, 5:1:3:3:4 vols

IR (Neat) 3000—3500 (broad), 1720, 1680

NMR ($CDCl_3$): 7.95 (bm, 4H), 5.35 (m, 4H) 1.4—2.6 (m, 14H), 1.1—1.7 (m, 10H) 0.06 (t (J=7H), 3H)

Since the free amine was unstable the di-N-acetyl derivative (27) was used for analysis, and it was obtained by acetylation with acetic anhydride in pyridine:

Anal. ($C_{33}H_{54}N_2O_8$) C,H,N.

The compound *26* was synthesized by the above methods as a radioactive derivative on a microscale (20 mg). All the radioactivity in the compound was present as $^{14}C$ in the GABA moiety of the compound. The product had a specific activity of 139 Ci/mmol and migrated with a $R_f$ 0.77 (BuOH, AcOH, pyridine, $H_2O$ 5:1:3:3:4) on TLC plates.

1,2-dilinolenoyl-3-(gamma-aminobutyroyl)propane-1,2,3-triol

A solution of 1,2-dilinolenoyl-3-(N-gamma-t-BOC aminobutyroyl)propane-1,2,3-triol (Compound 25) (40 mg, 0.05 mmol) in 15 ml of methylene chloride containing 0.25 ml of trifluoroacetic acid at 0°C was stirred overnight under $N_2$. The solution was then concentrated under vacuum. The viscous residue was passed through a silica gel (8 g) column, eluting first with ethyl acetate:petroleum ether (1:1 vols) and then with chloroform:methanol (80:20 vols). The ninhydrin positive (TLC) fractions of the chloroform/methanol eluents were combined and concentrated to obtain 62.1 mg of material. This was dissolved in 15 ml of chloroform and treated with 10 ml of $NaHCO_3$ solution containing sodium chloride. The chloroform layer was separated, dried over anhydrous $Na_2SO_4$ and concentrated to yield 29.5 mg (45 μmol) (84%) of pure product (Compound *28*). The characteristics of the product were as follows:

TLC: $R_f$ 0.58 in chloroform:methanol, 9:3 vols

IR (Neat) 3500, 1730, 1675

NMR ($CDCl_3$): 5.35 (m, 12H, vinyl H) 4.22 (m, 5H, glycerol H) 2.70 (M, 2H), 2.41—1.37 (bm, 14H) 1.25 (bs, 12H), 0.87 (t (J=7.5Hz), 6H)

Since the free amine was not stable the N-acetyl derivative (Compound *29*) was prepared for analysis by reacting the free amine with acetic anhydride and 4-dimethylaminopyridine.

Compound *29* was also synthesized as a radioactive derivative using the above methods on a microscale. The product had all its label as $^{14}C$ in the GABA substituent of the molecule. It migrated at an $R_f$ = 0.58 in ($CHCl_3$, $CH_3OH$, 9:3) on TLC, and had a specific activity of 41 μCi/mmol.

1-(gamma-carbobenzoxyaminobutyroyloxy)2,3-O-isopropylidenedioxy propane (Compound 30)

Isopropylidene glycerol (Compound *20*) (370 mg, 2.8 μmol), gamma-carbobenzoxyamino-butyric acid anhydride (1.28 g, 2.8 μmol) and 4-dimethylaminopyridine (360 mg, 3 mmol) were dissolved in 20 ml of dry benzene and the solution was stirred overnight at room temperature under anhydrous conditions. The solution was washed with 5% sodium bicarbonate solution, 0.1N HCl solution and water. The organic layer was dried over anhydrous $MgSO_4$ and concentrated in vacuum to yield 849 mg (2.4 μmol) (yield 86%) of product (Compound *30*). The characteristics of Compound *30* were as follows:

TLC: $R_f$ 0.57 ethyl acetate:hexane, 1:1

IR (Neat) 3350, 1710, 1700

NMR ($CDCl_3$): δ 7.38 (s, 5H, aromatic H) 5.14 (s, 2H, benzyl $CH_2$) 4.7—5.05 (bm, 1H, N*H*) 3.60—4.50 (m, 5H, glycerol CH,$CH_2$) 3.25 (m, 2H), 2.40 (t (J=11Hz) 2H) 1.86 (m, 2H), 1.45 (s, 3H), 1.38 (s, 3H)

Anal: ($C_{18}H_{25}NO_6$) C,H,N.

1-(gamma-aminobutyroyl)propane-1,2,3-triol (Compound 31)

A solution of the carbobenzoxyl derivative (Compound *30*, 200 mg, 570 μmol) and 10% palladium on charcoal (30 mg) in 10 ml of ethanol was stirred overnight under $H_2$. The solution was filtered to remove the catalyst and was concentrated under vacuum to obtain the product (Compound *31*) as a viscous liquid (104 mg, 432 μmol) (yield 76%) which had a single ninhydrin-positive spot on analytical thin-layer chromatography. The characteristics of the compound were as follows:

TLC: $R_f$ 0.35 in chloroform:methanol, 3:9

IR (Neat) 3500, 1720

NMR ($CDCl_3$) 6.8 (bs 3H), 4.4—3.55 (m, 5H)

(acetate salt) 2.85 (t, 3H), 2.43 (m, 2H) 1.95 (s, 3H), 1.4 (s, 3H) 1.35 (s, 3H), 1.2 (m, 2H)

The product obtained after hydrogenolysis (150 mg, 690 μmol) was taken up in 20 ml of methylene chloride, and 0.2 ml of trifluoroacetic acid was added and the mixture was stirred at room temperature for 3 hr. The solvent was removed under vacuum, and the residue was passed through a silica gel column, eluting with chloroform:methanol to obtain 77.7 mg (267 μmol) of the trifluoroacetate (yield 39%). The characteristics of the compound were as follows:

TLC $R_f$ 0.45 in BuOH:AcOH:$H_2O$, 4:1:1

IR (Neat): 3500, 1670

NMR δ 41—3.05 (mg, 7H),

$CD_3COCD_3$) 2.53 (t (J=7Hz, 2H) 2.15 (m, merges with acetone peak) 1.55—1.05 (m, 2H)

A radioactive derivative (specific activity 645 μCi/mM) was synthesized using the above procedures with CBZ-$^{14}C$(U)-GABA anhydride.

The overall yields with which some of the final products were obtained, as well as the specific activities of the corresponding $^{14}C$ compounds labeled in the GABA radical are shown in Table I, hereinbefore. The results of the elemental analysis of compounds 21, 23, 24, 25, 30, 27 and 29 are shown in Table II, hereinbefore.

Melting points were determined on a hot stage Fisher Jones apparatus and are uncorrected. IR spectra were recorded on a Perkin-Elmer Infracord Spectrophotometer and are reported in $cm^{-1}$. NMR spectra were recorded on a CFR 20 Spectrometer. Chemical shifts are reported in parts per million with tetramethylsilane as the internal reference. The multiplicities are expressed as follows: s = singlet, d = doublet, t = triplet, q = quartet, m = multiplet, b = broad. Elemental analyses were performed by the Midwest Microlab Ltd., Indianapolis, IN, and were in agreement (within ± .4%) with the proposed structure. Thin-layer chromatography (TLC) was performed on 100-μm-thick precoated silica gel chromatogram sheets (Eastman).

Biological Studies with Compounds of Example 3

Brain Uptake Studies

The capacity of some of the compounds of Example 3 to penetrate the blood-brain barrier of test mice was evaluated by measuring their brain penetration index (BPI). Table VI lists the results obtained. The BPI value for GABA is 1.0%. This is increased by 90-, 127-, and 3-fold, respectively, for Compounds *26, 28,* and *31.* Thus, there seems to exists no blood-brain barrier for the lipid derivatives. In fact, the uptake into brain for Compound *28* is higher than in liver, suggesting that there is a preferential accumulation into the CNS. Studies of the time course of uptake indicate that the brain continues to accumulate Compounds *26* and *28,* even at 3 and 4 hr after the s.c. injection, while liver levels reach a maximum at 45—60 min, and then decrease. This pattern is presumably due to the slow secondary release of the highly insoluble molecules into the bloodstream from lipoid tissue stores or late absorption from the site of injection. Due to the similarity of the test compounds to natural components of membrane lipids (especially Compound *28*), it seems probable that the compounds may become associated with brain membrane lipid bilayers to provide a reservoir for release of GABA by hydrolysis by the esterases that may be present in the CSF and brain membrane. Thin-layer chromatographic (TLC) analysis of tissue homogenates showed that 60% of the original Compound *26* was present in brain as the intact molecule at 4 hr after the injection, whereas the liver and kidney of the same animal had only 30% and 10% of the radioactivity present as *26.* The remainder of the label associated with each tissue was present mainly as low molecular weight volatile products (probably acetic acid) in the liver and kidney.

TABLE VI: UPTAKE OF LIPID ESTERS OF GABA

| COMPOUND | NO. | DOSE (µmol/kg) | TIME (min) | (BRAIN) (nmol/g) | (LIVER) (nmol/g) | BPI (%) |
|---|---|---|---|---|---|---|
| GABA (G) | | 270 | 5 | 1.03 | 104 | 1±.09 |
| glyceryl-GABA | 31 | 270 | 5 | 4.8±0.2 | 157±12 | 3±0.35 |
| | | 270 | 15 | 14 | 112 | 12 |
| | | 270 | 30 | 16.4 | 91 | 18 |
| linolenyl-diGABA-glyceride | 26 | 188 | 5 | 3.0±0.2 | 3.3±0.2 | 90±13 |
| | | 200 | 60 | 2.4 | 2.9 | 87 |
| | | 360 | 180 | 9.4 | 5.5 | 170 |
| di linolenoyl GABA-glyceride | 28 | 67 | 5 | 5.9±0.4 | 4.7±0.4 | 127±8.3 |
| | | 20 | 60 | 1.4 | 3.1 | 44 |
| | | 170 | 60 | 6.0 | 8.8 | 68 |
| | | 60 | 240 | 7.1 | 18.3 | 39 |

BPI = Brain Penetration Index, as defined hereinbefore. Each compound was tested in Balb/c mice (20 ± 2 g), as a solution in 0.5 ml of 25% (v/v) of propylene glycol in water. All the 5-min BPI values are the means ± S.E.M. for three measurements.

Measurement of Brain Penetration Index

Each of the $^{14}C$-labeled Compounds *26, 28* and *30* were in 125 µl of the propylene glycol diluted with water up to 0.5 ml, and then injected subcutaneously into test Balb/c mice (20 ± 2 g weight). After 5 min the animals were sacrificed and the brain and liver were removed, weighed and homogenized in 8 ml and 10 ml of brain protein solvent, respectively. Next 0.5 ml aliquots were mixed with 10 ml of Aquasol 2 (New England Nuclear Corp., Boston, MA) and counted in a Beckman liquid scintillation counter. The data for the label in each tissue were used to calculate the total quantity of the compound present in brain and liver per gram of tissue. The ratio of the amount in brain as a percent of that present in liver at 5 min was taken as the Brain Penetration Index (BPI). Measurements at longer uptake times were also obtained to evaluate the accumulation of the compounds in the brain. The results of these tests are shown in Table VI hereinbefore.

Pharmacological Properties

The pharmacological properties of the lipid esters of GABA were evaluated by two methods.

(1) General Locomotor Activity

The general locomotor activity of mice was measured in the Stolting activity monitor apparatus during 60 min after an intraperitoneal (i.p.) injection of a test compound. Dose-response curves were obtained, and the dose at which behavioral activity was reduced by 50% ($ED_{50}$) was taken as a measure of the effectiveness of each compound. GABA itself had no significant effect even at a dose of 9.7 mmol/kg (1 g/kg) whereas Compounds *26* and *28* were active at a much lower dose ($ED_{50}$ = 69 and 43 mol/kg, respectively). The results are shown in Table VII.

TABLE VII: PHARMACOLOGICAL TESTING OF LIPID ESTERS OF GABA

| COMPOUND | $ED_{50}$ (INJECTED DOSE) (µmol/kg) |
|---|---|
| GABA | >9700 x $10^3$ [a] (inactive) |
| 26 | 69 |
| 28 | 43 |
| 31 | inactive |

The $ED_{50}$ data were obtained from dose-response measurements using 8 animals per dose. The changes are the 60-min general motor activity values as compared to vehicle-injected and uninjected controls.

[a] The value for GABA was obtained for a 10-min time span after the injection; no significant differences from controls were observed for a 60-min time span.

Measurements of the actual concentrations of the molecules present after 5 min in the brain at these doses by TLC indicated that *26* and *28* were present (at $ED_{50}$) at the level of 2.1 and 1.7 nmol, respectively per gram of brain tissue. The diGABA lipid analogue, Compound *26*, also had the curious property of enhancing locomotor activity at doses below 10 mg/kg and depressing such behavior at high doses ($ED_{50}$ = 36 mg/kg) as is shown in Figure 2. This result is similar to the reported effects of Baclofen (p-chlorophenyl GABA). The monoGABA ester of glycerol (Compound *31*) was water soluble and showed insignificant pharmacological activity.

(2) Effect on bicuculline-induced seizures

The effect of the compound to delay the onset of a bicuculline-induced seizures was evaluated. Here, both Compounds *26* and *28* had only weak anticonvulsant properties when they were administered at doses of 30 mg/kg (50 µmol/kg). These results suggest that the natural lipid esters of GABA have some sedative properties but lack substantial anticonvulsant effects.

(3) Effect on stereotypy behavior

Stereotypy tests were carried out by the method of *A. Campbell et al*, Neuropharmacol. 21, 953 (1982). Test mice were pretreated with Compound *26* (i.p. injection), then 15 min later apomorphine or D-amphetamine sulfate was injected i.p. The animals were observed every 10 min and rated for stereotypic behavior in comparison to controls.

Rating scale:

0 = no stereotypy
1 = discontinuous sniffing
2 = continuous sniffing
3 = continuous sniffing and jaw movements

The average total score per animal was obtained (maximum positive score = 18) over a 60-min period. A compound which depresses this score is considered to have properties of a neuroleptic with a potential for

use as an antipsychotic agent. The results (see Table VIII) show a pronounced reduction of stereotypy, down to 24% of control.

TABLE VIII. STEREOTYPY DATA

| Induced By<br>Dose | Apomorphine<br>(1 mg/kg) | D-Amphetamine Sulfate<br>(3 mg/kg) |
|---|---|---|
| Vehicle control | 15.3 ± 3 | 13.8 ± 1.5 |
| Compound 26 (170 μmole/kg) | 8 ± 3.6 | 3.3 ± 0.9 |
| Stereotypy (% of control) | 52 | 24 |

Number of animals per test group = 4

Example 4

Lipid/Water Distribution of some Ester Derivatives of GABA

The lipid/water distribution of some of the compounds from Examples 2 and 3, as well as for GABA itself were determined by measuring the distribution of the compounds in a mixture of n-octanol and water.

Measurement of the n-Octanol/Water Partition Coefficient

The n-octanol/water partition coefficients were obtained from measurements of the distribution of 1, 3, 5 and 6 μmol quantities of each $^{14}C$-labeled compound into a mixture of 5 ml of n-octanol and 5 ml water. At each concentration, the amount of labeled compound in the aqueous and n-octanol phases was calculated from the amount of radioactive material present after stirring for 18 hours. A plot of the amount in the n-octanol versus that in the aqueous phase gave a straight line with a slope of K, the partition coefficient.

$$K = \text{partition coefficient} = \frac{\text{A n-octanol phase}}{\text{A aqueous phase}}$$

where A = the molar concentration of the compounds.

The data showing the lipid/water partition coefficients for the derivatives of GABA are shown in Table IX. Results from the experiments with the same ester derivatives of GABA described in Examples 1, 2 and 3 are also incorporated therein. The procedures followed for the determination of the effect of the compounds on the *in vivo* time of onset of bicuculline-induced seizures and on the *in vivo* inhibition of general locomotor activity are as described in the previous examples. The values for the brain penetration index (BPI) shown in Table I have also been incorporated into Table IX for comparative purposes.

TABLE IX: LIPID/WATER PARTITION COEFFICIENTS

| COMPOUND | BPI[f] | $K^a_{OW}$ | In Vivo[b] | In Vivo[c,e] |
|---|---|---|---|---|
| GABA | 1 | 0.004 | 0 | 0 |
| Glyceryl-GABA | 3 | 0.01 | 0 | 0 |
| Inositol-tri GABA | 36 | n.d.[g] | n.d. | 4 |
| Dexamethasone-GABA | 81 | 0.9 | 0 | 0 |
| Inosital-GABA | 11 | n.d. | n.d. | 0 |
| 3-Glucosyl-GABA[d] | 104 | 0.21 | 0 | 0 |
| Butyl-GABA | 74 | n.d. | 1 | n.d. |
| Linolenoyl-GABA | 2 | n.d. | 0 | n.d. |
| diGABA-linolenoyl glyceride | 90 | 2.1 | 4 | 4 |
| GABA-dilinolenoyl glyceride | 127 | 6.6 | 5 | 4 |
| Cholesteryl-GABA | 25 | 110. | 5 | 5 |

a - $K_{OW}$: Octanol/Water Partition Coefficient.
b - *In Vivo* Delay of Time of Onset of Bicuculline-induced seizures.
c - *In Vivo* Inhibition of General Motor Activity.
d - The Hydroxyl group at position 1 is a mixture of the and isomers.
e - 1-5: active, with 5 most active
0 : inactive
f - BPI: Blood Penetration Index as defined herein before.
g - n.d.: not done

The results of Table IX clearly distinguish between two groups of compounds. The first group is formed by compounds such as the cholesteryl ester of GABA which penetrate the blood-brain barrier and show biological activity in the present tests. The second group comprises compounds such as the glyceryl ester of GABA, which, although crossing the blood-brain barrier, do not show biological activity when adminstered at the indicated doses in the tests.

Example 5

Pharmacological studies with Compound 5 (linolenoyl ester of GABA) and Compound 6 (cholesteryl ester of GABA)

(1) Effect on bicuculine-induced seizures

The ability of a substance to delay the onset of bicuculline-induced epileptic seizures in the mouse was measured (Table X). There were no significant effects at 1 or 6 hr. At 24 hr after i.p. injection of the cholesteryl derivative (*6*) at a dose of 30 mg/kg (64 μmol/kg), the onset of seizures was delayed by 30% (as compared to saline controls) after an acute i.p. injection of bicuculline at 0.3 mg/kg. In contrast, the linolenyl ester of GABA had the surprising property of accelerating the seizure onset time by 60% at a dose of 50 mg/kg, even though it had no significant effect on the general motor activity of mice. The dexamethasone and butyl esters had no effect on seizures in mice.

The fact that the bicuculline-induced seizures could be influenced at 24 hr after the administration of two GABA ester (*5* and *6*) but not at earlier times indicates that no significant direct, acute anticonvulsant activity can be ascribed to the compounds.

TABLE X. ANTICONVULSANT PROPERTIES IN THE MOUSE

| Compound | Dose mg/kg | Onset of seizures: % Saline Control [a] | Survival Time: % Saline Control |
|---|---|---|---|
| saline | | 100 | 100 |
| vehicle | | 109 ± 7 | 123 ± 10 |
| cholesterol | 84 | 118 ± 6 | 110 ± 7 |
| 6 | 3 | 120 ± 6* | 95 ± 15 |
| 6 | 16 | 129 ± 4** | 101 ± 20 |
| 6 | 30 | 130 ± 8** | 104 ± 18 |
| 6 | 84 | 137 ± 7*** | 109 ± 9 |
| 5 | 12 | 102 ± 4 | 118 ± 10 |
| 5 | 17 | 73 ± 15** | 111 ± 14 |
| 5 | 25 | 78 ± 9** | 68 ± 9*** |
| 5 | 36 | 50 ± 5*** | 62 ± 6*** |
| 5 | 50 | 40 ± 11*** | 63 ± 8*** |

[a] Anticonvulsant action at 24 hr after pretreatment with the test compound. The seizure onset time was measured in seconds following an injection of bicuculline (0.3 mg/kg). Data for saline controls were 184 ± 19 and 253 ± 28 s, respectively, for onset of seizures and death with respect to *5*. Data are means plus or minus SEM, n ≥ 6 in each group. * = $p < 0.5$; ** = $p < 0.05$; *** = $p < 0.005$ by t test of difference between drug and saline controls.

(2) Measurement of activity of intact esters

The above results demonstrate that a compound such as cholestrol, which readily passes through the blood-brain barrier, can transport covalently bound GABA across the barrier. Once such a compound enters the CNS, its pharmacological activity could be due to the properties of the intact molecule or to its capacity to release GABA after hydrolysis. To test for the possibility that the intact molecules might be active, the capacity of Compounds *5* and *6* to inhibit the binding of [$^3$H]GABA to GABA "receptors" using membrane preparations of rat cerebellum were measured. $IC_{50}$ values were determined by fitting data to linearized transformations, based on including at least two concentrations of antagonists above and below the $IC_{50}$, with at least triplicate replication per concentration. The results (see Table XI) indicate that the concentrations of Compounds *5* and *6* required for 50% inhibition ($IC_{50}$) of binding were much greater than for GABA. Both Compounds *6* and *7* had low binding affinities to the receptors in comparison with potent GABA agonists, which can displace [$^3$H]GABA at low nanomolar concentrations. There results suggest that the pharmacological activity of the cholesteryl ester of GABA (*6*) is probably not due to its direct interactions at GABA-receptor binding sites; its activity might, however, be related to release of GABA by hydrolysis of the ester as a "prodrug".

TABLE XI. INHIBITION OF [$^{3}$H]GABA BINDING TO SYNAPTIC MEMBRANES FROM RAT CEREBELLUM

| COMPOUND | $IC_{50}$,[a] nM |
|---|---|
| GABA | 70 |
| *5* | 40,000 |
| *6* | 10,000 |

[a] The $IC_{50}$ value represents the dose of each compound required for 50% inhibition of the binding of [$^{3}$H]GABA (15 nM) to synaptic membrane preparations (from rat cerebellum) in a receptor-binding assay.

(3) Enzymatic hydrolysis of cholesteryl [$^{14}$C] GABA ester by rat brain homogenate

Cholesterol hydrolases and esterases are present in mammalian brain tissue. That such enzymes can release GABA from Compound *6* was demonstrated (see Table XII) with the crude supernatant (10,000 g, $S_1$) fraction of rat brain homogenates as a source of hydrolases.

TABLE XII. ENZYMATIC HYDROLYSIS OF CHOLESTERYL [$^{14}$C]GABA ESTER BY RAT BRAIN HOMOGENATE

| Time/Min | Amount of Unreacted[a] Substrate *(6)*, nmol |
|---|---|
| 10 | $53.0 \pm 7.0$ |
| 30 | $43.5 \pm 6.3$ |
| 60 | $34.5 \pm 4.9$ |
| Control [b] | $55.6 \pm 2.6$ |

[a] Cholesteryl-[$^{14}$C]GABA ester *(6)* (3 mg/ml) was incubated at 37°C in the presence of $S_1$ fractions of rat brain homogenates (0.8 mg/ml of protein). Analysis of 40 µl aliquots removed at various time intervals was carried out, after quenching the reaction with 0.5 ml of ethanol, by thin-layer chromatography (TLC) on silica gel in $CHCL_3$/MeOH/HOAc (18:6:1).

[b] Control, the initial amount of substrate used, which did not change after 1 hr at 37°C.

(4) Inhibitory effect of cholesteryl GABA (C—G) Ester on evoked activity in rat hippocampal slices

Cholesteryl gamma-aminobutyrate (C—G) produces a dose-dependent inhibition of orthodromically-evoked discharge of CAI pyramidal cells. The duration of this inhibition is an order of magnitude longer than that produced by similar doses of GABA. The inhibitory effect of C—G is antagonized by picrotoxin, by low chloride incubation medium, and by an esterase inhibitor. These observations are consistent with the notion that C—G has GABA-mimetic actions in the CNS, and that this property is dependent upon release of GABA from the compound by hydrolysis by esterases present in the tissue. Thus, C—G is a "prodrug" for delivery of GABA to the CNS.

Methods

Transverse slices of hippocampus (350—375 μm thick) were prepared from Sprague-Dawley rats (200—300 g) and transferred onto a nylon net fixed over a 1.5 ml superfusion chamber. The slices were maintained at 32—33°C in a humidified atmosphere of 95%/5% $O_2/CO_2$. The standard medium consisted of (in mM): NaCl, 125; KCl, 3.5; $CaCl_2$, 2; $MgSO_4$, 1.5; $NaH_2PO_4$, 1; $NaHCO_3$, 24; and glucose, 10. Low chloride medium was prepared by substituting sodium isethionate for sodium chloride.

Extracellular potentials were recorded with NaCl-fitted micropipettes, and stimulus pulses were applied through bipolar platinum wire electrodes. The various drugs were dissolved in fresh medium (without glucose and phosphate) and applied to the surface of the tissue by pressure ejection from a micropipette, *Sakai, Swartz and Woody,* (1979).

The inhibitory effectiveness of various compounds was investigated by measuring their ability to suppress the extracellular population spike recorded from the hippocampal CAI pyramidal layer and elicited by stimulation of stratum radiatum axons. The amplitude of this monosynaptic population spike is essentially proportional to the number of pyramidal cells discharging in response to a stimulus volley, *Andersen, Bliss and Skrede,* (1971). These pyramidal cells are normally inhibited by GABA interneurons through a circuit which is largely recurrent and terminates extensively, but not exclusively, on the pyramidal cell bodies. Thus, application of compounds with GABA-like activity to the CAI pyramidal layer mimics a normal inhibitory process, tends to suppress pyramidal cell discharge, and reduces the amplitude of the evoked population spike, *Alger, Jahr and Nicoll* (1981); *Anderson, Dingledine, Gjerstad, Langmoen and Mosfeldt Laursen* (1980); *Knowles and Schwartzkorin* (1981); *Schwartzkroin and Knowles* (1983).

The various compounds were applied as droplets to the pyramidal layer approximately 75 μm from the recording electrode. The volume of a single drug application was about 300 picoliters. This droplet size was small enough to avoid any artifactual effects on the stimulating electrode, *Langmoen, Segal and Andersen* (1981). The volume ejected from each pipette was calibrated prior to use by measuring the diameter of the ejected droplet and adjusting the duration of the pressure pulse (usually 100—500 msec) until a standard diameter was reached. A constant droplet volume was used throughout the experiments. Where dose-response information was required, several pipettes with different drug concentrations were used. This method gave reproducible results, as indicated by the fact that different pipettes with the same drug concentration produced similar results.

Results

Droplets of GABA applied to the CAI pyramidal layer produced rapid, dose-dependent inhibition of the evoked population spike. The maximum inhibitory effect was evident within a few seconds of droplet application, and recovery was usually complete within about 2—3 min. The effect could be repeated at 5- to 10-min intervals with little or no adaptation. Application of muscimol also produced dose-dependent inhibition of the CAI population spike. The doses required (i.e., the concentration in the drug pipette) were nearly a thousand-fold lower than that of GABA for effects of comparable magnitude. In addition, the duration of the muscimol effect was roughly 100 times longer than that of a comparably effective dose of GABA.

In some experiments the extracellular synaptic potential was recorded from stratum radiatum, and the stimulation intensity was adjusted until no population spike was evoked. Under these conditions both GABA and muscimol applied to stratum pyramidale reduced the amplitude of this potential. This result is consistent with the large conductance increases in pyramidal cells associated with GABAergic synaptic action at the soma, *Dingledine and Langmoen* (1980), *Alger et al* (1981), *Andersen et al* (1980).

Addition of picrotoxin (10 μM) to the medium or replacement of the standard medium with low chloride medium significantly attenuates GABA-mediated inhibition in hippocampal slices, and a single stimulus pulse produces multiple population spikes, *Ogata* (1975), *Corrigall and Linseman* (1980), *Alger et al* (1981). Under these conditions, GABA and muscimol had little effect on the amplitude or number of evoked population spikes.

Application of cholestryl γ-aminobutyrate (C—G) to the CAI pyramidal region of hippocampal slices also resulted in dose-dependent inhibition of the evoked population spike. The maximum magnitude of the C—G effect was less than that of a similar dose of GABA; however, the duration of the effect was roughly an order of magnitude longer. In the experiment, recovery of the population spike amplitude to 90% of the pre-droplet control value after an approximately half-maximal dose of C—G required about 14 min, while the recovery time after a GABA droplet was only 1—2 min.

Although C—G clearly produces a prolonged suppression of hippocampal pyramidal cell evoked

discharge, the mechanism may be unrelated to GABA-mediated inhibition. If C—G acts by a GABAergic mechanism it should be antagonized by picrotoxin and by low chloride medium. Picrotoxin (10 μM) added to the medium did essentially eliminate the C—G effect. This antagonism was reversible; after washout of the picrotoxin, C—G was as effective as it was in the initial application. Replacing the standard medium with low chloride medium produced a similar result which was also reversed when standard medium was reintroduced. The results for the low chloride condition were obtained with C—G dissolved in low chloride medium. When C—G was dissolved in standard medium and applied to slices in low chloride medium, there was a small inhibitory effect which lasted no more than about a minute. This effect was presumably due to the chloride applied to the tissue along with the C—G. This observation emphasizes the chloride-dependent nature of the C—G inhibitory effect.

To test the possibility that the GABA-like actions of C—G are dependent on *in situ* enzymatic hydrolysis of the compound, experiments were conducted with phenylmethylsulfonylfluoride (PMSF) added to the slice incubation medium. PMSF is a potent, irreversible inhibitor of a variety of esterases, *Gold* (1967), and *in vitro* experiments with brain homogenate show that PMSF significantly slows the degradation of C—G, *Shashoua et al* (1984). Although PMSF is toxic, hippocampal slices can be incubated in 0.5 mm concentration for over 2 hr before showing significant loss of physiological activity, and if PMSF is removed after about 1 hr of incubation the slices appear to be physiologically stable for at least an additional 2 hr. When slices were incubated in PMSF (0.5 mM) for about 45 min, the inhibitory effect of C—G was significantly reduced. Washout of PMSF for about 1 hr did not reverse this effect. The effectiveness of GABA showed no change with PMSF, although recovery times seemed to be slowed somewhat. Thus, it appears that some esterase activity is essential to the physiological actions of C—G, but not to those of GABA.

All the compounds having an n-octanol/water partition coefficient greater than 0.9 were shown to be active both in the *in vitro* electrophysiological measurements of inhibitory effects on brain slices and in the *in vivo* inhibition of general motor activity. This type of result is consistent with the hypothesis that some lipid solubility must be present to allow the compounds to get into the cell membrane and interact with the GABA receptors.

Example 6

Synthesis of cholesteryl gamma-vinyl gamma-aminobutyrate hydrochloride (Compound 33)

A. Cholesteryl gamma-vinyl-N-gamma-t-BOC-aminobutyrate (Compound 32)

A solution of cholesterol (80 mg, 0.206 mmoles), gamma-vinyl-N-t-BOC GABA (40 mg, 0.197 mmoles), dicyclohexyl carbodiimide (40 mg) and 4-dimethylaminopyridine (3 mg) in 20 ml of methylene chloride was stirred overnight at room temperature. The precipitated dicyclohexyl urea was removed by filtration. The filtrate was washed with 0.1N HCl, 5% $NaHCO_3$ solution and brine. The organic layer was dried over $Na_2SO_4$ and concentrated, and the residue was purified by column chromatography over silica gel eluting with ethyl acetate and petroleum ether. Yield: 84 mg (75%) of Compound *32*.

MP 114—115°C.

*Analysis:*

| | | | |
|---|---|---|---|
| Calc. for $C_{38}H_{63}NO_4$: | C, 76.38; | H, 10.55; | N, 2.35. |
| Found: | C, 76.57; | H, 10.12; | N, 1.86. |

*IR* (Nujol): 3360, 1725, 1690, 1175 $cm^{-1}$.

B. Cholesteryl gamma-vinyl gamma-aminobutyrate hydrochloride (Compound 33)

A solution of cholesteryl gamma-vinyl-N-gamma-t-BOC aminobutyrate (Compound *32*) (0.39 g, 0.65 mmoles) in 15 ml dioxane and 2 ml 4 m HCl-dioxane was stirred for 3 hr at room temperature. The product was precipitated as a white solid which was filtered and washed with ethyl acetate to get 244.5 mg (0.46 mmoles) of the hydrochloride.

*IR* (Nujol): 3500, 1725, 1605, 1510 $cm^{-1}$.

Example 7

Synthesis of gamma-vinyl GABA glucose-3-ester (Compound 35)

A. (1,2:5,6-diisopropylidene-D-glucose-3) gamma-vinyl-N-gamma-t-BOC gamma-aminobutyrate (Compound 34)

A solution of 1,2:5,6-diisopropylidene-D-glucose (215 mg, 0.83 mmoles), gamma-vinyl-N-gamma-t-BOC amino butyric acid (gamma-vinyl BOC GABA) (166 mg, 0.73 mmoles), dicyclohexyl carbodiimide (170 mg) and 4-dimethylaminopyridine (12 mg) in 10 ml of methylene chloride was stirred at room temperature for 4 hr. The reaction mixture was filtered to remove dicyclohexyl urea (146 mg) that was precipitated during the reaction. The filtrate was concentrated to get 344 mg of crude product which was purified by passing through a small silica gel column with ethyl acetate and petroleum ether. Yield: 244 mg (71%) of Compound *34*.

*IR* (Neat): 3450, 1730, 1690, 1640, 1510, 1360 $cm^{-1}$.

*Analysis:*

| | | | |
|---|---|---|---|
| Calc. for $C_{23}H_{37}NO_9$: | C, 58.60; | H, 7.86; | N, 2.97. |
| Found: | C, 58.53; | H, 8.02; | N, 2.80. |

B. Gamma-vinyl GABA glucose-3-ester (Compound 35)

A mixture of (1,2:5,6-diacetone-D-glucose-3) gamma-vinyl-N-gamma-t-BOC aminobutyrate (Compound *3*) (70 mg, 0.148 mmoles) in 4 ml of ethanol and 2N HCl (8 ml) was stirred overnight initially for 5 hr at room temperature and then at 5°C. The mixture was concentrated. The residue was taken in water and was filtered. This was concentrated and co-evaporated with ethanol to remove final traces of water. Yield: 45 mg (0.13 mmoles, 88%) of Compound *35*.

TLC: $R_f$: 0.28 (propanol, acetic acid, water 8:1:1) (silica gel).

*IR* (Neat): 3400, 1725, 1625 $cm^{-1}$.

Example 8

Synthesis of 1-linolenoyl 2,3-di(gamma-vinyl-gamma-amino butyryl) glycerol, dihydrochloride (Compound 37)

A. 1-Linolenoyl 2,3-di(gamma-vinyl-N-gamma-t-BOC aminobutyryl) glycerol (Compound 36)

A solution of linolenoyl glycerol (31 mg), gamma-vinyl BOC GABA (71 mg), dicyclohexyl carbodiimide (36 mg) and 4-dimethylaminopyridine (18 mg) in 10 ml of methylene chloride was stirred overnight at room temperature under nitrogen atmosphere. The mixture was filtered and the filtrate was washed with 0.1N HCl, 5% $NaHCO_3$ solution and brine. The organic layer was dried over anhydrous sodium sulfate and was concentrated to get a residue. This was purified by column chromatography on silica gel eluting with ethyl acetate and petroleum ether, to yield 53 mg of the product, Compound *36*.

B. 1-Linolenoyl 2,3-di(gamma-vinyl gamma-aminobutyryl) glycerol, dihydrochloride (Compound 37)

A solution of the di-BOC derivative (Compound *36*) (16 mg) and 0.2 ml of HCl in dioxane (3 M) in 3 ml of methylene chloride was stirred under nitrogen atmosphere at room temperature for 3—4 hr. The product was purified by column chromatography on silica gel using chloroform and methanol. Yield: 12.6 mg of Compound *37*.

Example 9

Synthesis 1-linolenoyl-2-N-gamma-t-BOC aminobutyryl 3-gamma-vinyl gamma-aminobutyryl glycerol (Compound 40)

A. 1-Linolenoyl 2-gamma-vinyl-N-gamma-t-BOC aminobutyryl gloycerol (Compound 38)

A solution of monolinolenoyl glycerol, 31 mg (0.088 mmoles), gamma-vinyl BOC GABA, 18 mg (0.079 moles), dicyclohexyl carbodiimide, 18 mg and 4-dimethylaminopyridine, 3 mg, in 5 ml of methylene chloride was stirred overnight at room temperature under nitrogen atmosphere. The reaction mixture was filtered and the filtrate was washed with 0.1N HCl (ice cold), 5% sodium bicarbonate solution and brine. The solution was dried ($Na_2SO_4$) and concentrated to get 47 mg (0.087 mmoles, 99%) of crude product which was purified by column chromatography on silica gel, eluting with ethyl acetate, petroleum ether, Compound *38*.

TLC: $R_f$: 0.6 (ethyl acetate, petroleum ether 1:1) (silica gel).

*Analysis:*

| | | | |
|---|---|---|---|
| Calc. for $C_{41}H_{68}N_2O_{10}$: | C, 65.78; | H, 9.09; | N, 3.74. |
| Found: | C, 65.82; | H, 9.80; | N 3.99. |

B. 1-Linolenoyl 2-N-gamma-t-BOC-aminobutyryl 3-N-gamma-t-BOC aminobutyryl glycerol (Compound 39)

A solution of the glycerol diester, 89 mg (0.166 mmoles), BOC—GABA anhydride, 90 mg and 4-dimethylaminopyridine, 22 mg, in 20 ml of benzene was stirred overnight at room temperature under nitrogen atmosphere. The mixture was worked up as before to get 115 mg (0.159 moles) of product. Yield 96%.

TLC: $R_f$: 0.6 (ethyl acetate, petroleum ether 1:1) (silica gel).

*Analysis:*

| | | | |
|---|---|---|---|
| Calc. for $C_{41}H_{68}N_2O_{10}$: | C, 65.78; | H, 9.09; | N, 3.74. |
| Found: | C, 65.82; | H, 9.80; | N, 3.99. |

C. 1-Linolenoyl 2-gamma-aminobutyryl 3-gamma-vinyl-gamma-aminobutyryl glycerol dihydrochloride (Compound 40)

A solution of the di-BOC-amino derivative (28 mg) and 4N HCl in dioxane (0.3 ml) in methylene chloride (4 ml) was stirred at room temperature for 3 hr. The mixture was concentrated in vacuum and the residue was purified on a silica gel column eluting with chloroform, methanol to yield 9 mg of the dihydrochloride, Compound *40*.

TLC: $R_f$: 0.45 (chloroform, methanol, acetic acid 9:3:0.5) (silica gel).

Examples 10—19

The cholesteryl esters of gamma-methyl-GABA, gamma-hydroxymethyl-GABA, gamma-propargyl-GABA, gamma-phenanthryl-GABA, gamma-hydroxy-GABA, gamma-phenyl-GABA, gamma-(1-chlorophenyl)-GABA, beta-oxo-GABA, alpha-propoxy-GABA and N-acetyl-GABA are prepared in accordance with Example 6, producing Compounds *41* through *50*, respectively.

Examples 20—29

The glucose-3 esters of beta-butyl-GABA, beta-hydroxy-methyl-GABA, beta-acetynyl-GABA, beta-hydroxy-GABA, gamma-phenyl-GABA, gamma-(1-methoxyphenyl)-GABA, beta-oxo-GABA, alpha-propoxy-GABA, N-pivalyl-GABA and N-pivalyl-alpha-methoxy-GABA are prepared according to Example 7, producing Compounds *51* to *60*, respectively.

Examples 30—39

The 1-linolenoyl-2,3-diGABA analogue triesters of glycerol, where the GABA analogues are respectively gamma-methyl-GABA, beta-methoxy-GABA, alpha-methoxy-beta-methoxy-GABA, alpha-(3-hydroxyphenyl)-GABA, gamma-methyl-vinyl-GABA, gamma-i-propyl-GABA, gamma-fluoromethyl-GABA, gamma-difluoro-methyl GABA, beta-anthracyl-GABA and N-acetyl-GABA are prepared in a manner similar to Example 8, producing Compounds *61* to *70*, respectively.

Example 40

Self-stimulation reward test

The procedure measured the capacity of a given pharmacological agent to inhibit rats from receiving stimulating currents provided by electrodes implanted in their brains (lateral hypothalamus), *Stellar et al,* Pharmacol. Biochem. & Behav., 18, 433—442 (1983). The animals will press a lever to receive a current from the implanted electrode in preference to all else. The rate at which they press the lever depends on the intensity of the current (reward) that is being produced by the electrode. The intensity of the current is varied by raising the frequency at which 250 A pulses (0.1 msec duration) are delivered during a 0.5 sec time span in response to a lever press. A plot of the log of the frequency of the delivered pulses vs. the rate of lever presses for an animal gives a self-stimulation reward curve. Injection of a drug which has a tranquilizing effect shifts such a curve to higher frequencies. The shift of this curve (measured at the halfway point) gives a parameter which is expressed as the "Depression of the Self-Stimulation Reward" or DSSR. The maximum DSSR value obtained for the most powerful neutoleptics and tranquilizing compounds tested so far, seldom exceeds 0.3. This is designated as 100%. In the actual tests, the maximum DSSR value obtained for a given dose of drug and the half-life time [duration half-life (DHL)] of its return to 0% (the control state) was measured.

Results of Self-Stimulation Reward Tests

| Compound | Dose (μm/kg) | Max. DSSR | DHL |
|---|---|---|---|
| I (LGVG)* | 16 | 90 | 30 min |
| II (CVG)** | 40 | 83 | 24-48 hr |
| gamma-vinyl GABA (control) | 31 | 0 | 0 |

* linolenoyl gamma-aminobutyryl gamma-vinyl gamma-aminobutyryl glycerol

** cholesteryl gamma-vinyl gamma-aminobutyrate

Having now fully described this invention, it will be appreciated by those or ordinary skill in the art that the same can be practiced with a wide and equivalent range of compositions, modes of administration, therapeutic treatments, and the like, without affecting the spirit or scope of the invention or any embodiment thereof.

**Claims**

1. A compound of the formula:

$$A(O-CO-\overset{R_3}{\overset{|}{C}}H-\overset{R_2}{\overset{|}{C}}H-\overset{R_1}{\overset{|}{C}}H-\overset{R_4}{\overset{|}{N}}H)_n$$

wherein $R_1$, $R_2$ and $R_3$ are the same or different and are selected from:
(a) hydrogen
(b) lower alkyl groups having one to four carbon atoms;
(c) substituted lower alkyl groups having one to four carbon atoms;
(d) lower alkenyl groups having one to four carbon atoms;
(e) substituted lower alkenyl groups having one to four carbon atoms;
(f) lower alkynyl groups having one to four carbon atoms;
(g) substituted lower alkynyl groups having one to four carbon atoms;
(h) aryl groups;
(i) substituted aryl groups;
(j) hydroxy groups or hydroxy groups protected with lower acyl or aroyl groups;
(k) lower acyl groups;
(l) oxo groups, in which case hydrogen is not present on the carbon atom;
(m) amino groups;
(n) substituted amino groups;
(o) $R_1$ and $R_3$ together forming a carbocyclic ring;
(p) $R_2$ and $R_3$ together forming a carbocyclic ring;
$R_4$ is hydrogen or acyl;
A represents the radical of a compound having at least one esterifiable OH group, selected from the group consisting of carbohydrates, saturated aliphatic alcohols with 4—40 carbon atoms having more than one OH substituent, unsaturated aliphatic alcohols, cyclic alcohols, aromatic alcohols, alcohols containing at least one heteroatom and glyceryl esters of fatty acids, and being capable of crossing the blood-brain barrier of an animal;
n can vary from 1 to the total number of esterifiable OH groups contained in A;
or pharmaceutically acceptable acid addition salts thereof,
said ester having a Brain Penetration Index greater than about 2%.

2. The compound of Claim 1, having a Brain Penetration Index greater than about 3%.
3. The compound of Claim 2 having a Brain Penetration Index greater than about 25%.
4. The compound of Claim 1, having an n-octanol/water partition coefficient greater than about 1.
5. The compound of Claim 4, having an n-octanol/water partition coefficient greater than about 2.
6. The compound of Claim 5, having an n-octanol/water partition coefficient greater than about 6.
7. The compound of Claim 1, wherein A represents a radical of a carbohydrate having a carbon chain of 3 to 20 carbon atoms.
8. The compound of Claim 7 wherein the carbon chain contains 3 to 7 carbon atoms.
9. The compound of Claim 7, wherein the carbohydrate has the general formula:

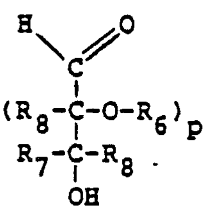

wherein:
(1) p is 1 to 18,
(2) $R_6$, $R_7$ and $R_8$ may be the same or different and represent:
(a) H;
(b) OH, except that $R_7$ and $R_8$ cannot be OH;
(c) branched or unbranched amino of the formula $(CH_2)_nNH_2$, wherein n is 1 to 15;
(d) branched or unbranched hydrazino of the formula $(CH_2)_nNHNH_2$, wherein n is as defined above;
(e) haloalkyl of the formula $(CH_2)_nCX_mH_{(3-m)}$, wherein n is as defined above and m is 1 to 3;
(f) alkyl of 1 to 15 carbon atoms;
(g) alkenyl of 1 to 15 carbon atoms;
(h) alkynyl of 1 to 15 carbon atoms; or
(i) $(CH_2)_nOPO_3H_2$, wherein n is as defined above; and in addition
(3) $R_7$ and $R_8$ may be the same or different and represent haloalkyl of the formula $CX_mH_{(3-m)}$ wherein m and X are as defined above; and
(4) $R_6$ represents acetyl.

10. The compound of Claim 5 wherein the carbohydrate is selected from the group consisting of D-glucose; D-glyceraldehyde; D-erythrose; D-threose; D-ribose; D-arabinose; D-xylose; D-allose; D-mannose; D-gulose; D-idose; D-galactose; D-talose; dihydoxyacetone; D-erythrulose; D-ribulose; D-xylulose; D-psikose; D-fructose; D-sorbose; their O-acetyl derivatives; their O-methyl derivatives; their deoxy-D-derivatives; their amino-D-derivatives; and their acid-D-derivatives.
11. The compound of Claim 1, wherein A represents the radical of a branched or unbranched saturated

aliphatic alcohol having more than one OH substituent and a carbon chain of 4 to 40 carbon atoms.

12. The compound of Claim 1, wherein A represents the radical of a branched or unbranched aliphatic alcohol, having at least one point of unsaturation.

13. The compound of Claim 12, wherein the carbon chain contains 4 to 40 carbon atoms.

14. The compound of Claim 13, wherein the carbon chain contains 4 to 30 carbon atoms.

15. The compound of Claim 14, wherein the carbon chain contains 8 to 20 carbon atoms.

16. The compound of Claim 12, wherein the alcohol is an alkenyl.

17. The compound of Claim 13, wherein the alcohol is an alkynyl.

18. The compound of Claim 5, wherein the alcohol has the formula:

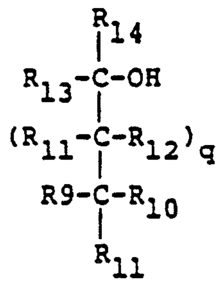

wherein:

(1) q is 1 to 38;

(2) $R_9$, $R_{10}$, $R_{11}$, $R_{12}$, $R_{13}$ and $R_{14}$ may be the same or different, and represent:

(a) H, with the proviso that at least one is different from H;

(b) OH, except that $R_{13}$ and $R_{14}$ cannot be OH;

(c) branched or unbranched amine of the formula $(CH_2)_nNH_2$, wherein n is 1 to 15;

(d) branched or unbranched alkyl of 1 to 15 carbon atoms;

(e) branched or unbranched hydrazino of the formula $(CH_2)_nNH—NH_2$, wherein n is as defined above;

(f) branched or unbranched haloalkyl of the formula $(CH_2)_nCX_mH_{(3-m)}$, wherein X represents F, Cl, Br, or I, n is defined as above, and m is 1 to 3;

(g) branched or unbranched alkenyl of 1 to 15 carbon atoms;

(h) branched or unbranched alkynyl of 1 to 15 carbon atoms;

(i) acetyl;

(j) their O-acetyl derivatives; and

(k) their O-methyl derivatives.

19. The compound of Claim 18, wherein the alcohol has one or more carbon rings, and $R_{10}$ and $R_{16}$ may be a cyclic structure containing one or more carbon rings.

20. The compound of Claim 19, wherein one or more of the carbon rings in the cyclic alcohol are aromatic.

21. The compound of Claim 20, wherein the aromatic alcohol is a polyalcohol.

22. The compound of Claim 1, wherein A represents a radical of a branched or unbranched, saturated or unsaturated cyclic alcohol, having a maximum of 18 ring carbon atoms.

23. The compound of Claim 22 having 5 to 14 carbon ring atoms.

24. The compound of Claim 22 having 6 to 10 carbon ring atoms.

25. The compound of Claim 22, wherein the cyclic alcohol is selected from the group consisting of cyclopentanol; cyclohexanol, cycloheptanol; cyclooctanol; cyclodecanol; cyclododecanol; cyclotetradecanol; cyclohexadecanol; cycloocatadecanol; inositol; their derivatives having multiple hydroxyl substituents; their O-acetyl derivatives; their O-methyl derivatives; their amine derivatives; and their halogenated derivatives.

26. The compound of Claim 22, wherein the cyclic alcohol may contain one or more aromatic rings.

27. The compound of Claim 1, wherein A represents the radical of a branched or unbranched alcohol with 4 to 40 carbon atoms having one or more heteroatoms.

28. The compound of Claim 27, wherein A contains from 1 to 3 atoms of oxygen, nitrogen or sulfur, or a combination thereof.

29. The compound of Claim 28, wherein A is selected from the group of alkyl, alkenyl and alkynyl alcohols having a carbon chain of 4 to 40 carbon atoms.

30. The compound of Claim 28, wherein the alcohol is a cyclic alcohol of 5 to 18 carbon ring atoms.

31. The compound of Claim 30 having 6 to 10 carbon ring atoms.

32. The compound of Claim 27, wherein the alcohol has one or more aromatic rings.

33. The compound of Claim 27, wherein the hetero alcohol is selected from the group consisting of ethanolamine, choline, serine, O-aminoacyglycerol, their alkyl derivatives, and their O-acyl derivatives.

34. The compound of Claim 1, wherein A represents the radical of a glycerol esterified with up to two radicals of carboxylic acids, which may be the same or different.

35. The compound of Claim 34, wherein the carboxylic acids, are branched or unbranched aliphatic acids.

36. The compound of Claim 35 having 10 to 30 chain carbon atoms.

37. The compound of Claim 35 wherein the carboxylic acids have an even number of carbon atoms.

38. The compound of Claim 34, wherein the carboxylic acids contain at least one point of unsaturation.

39. The compound of Claim 38, wherein the carboxylic acids are derived from an alkenyl or alkynyl radical having more than one point of unsaturation.

40. The compound of Claim 34, wherein the glycerol radical is esterified by one fatty acid and one GABA or GABA analogue.

41. The compound of Claim 40, wherein the glycerol radical is esterified by one fatty acid and two GABAs or two GABA analogues.

42. The compound of Claim 40, wherein the glycerol radical is esterified by two fatty acids and one GABA or GABA analogue.

43. The compound of Claim 35, wherein the carboxylic acids are selected from a group consisting of lauric acid, myristic acid, palmitic acid, stearic acid, arachidic acid, palmitoleic acid, oleic acid, linoleic acid, linolenic acid, arachidonic acid, cerebronic acid, cardiolypin, their O-acetyl derivatives, there O-methyl derivatives, and their amino derivatives.

44. The compound of Claim 1, wherein A is the radical derived from a compound selected from the group consisting of inositol, cholesterol, dexamethasone, dilinolenoyl glycerol, and linolenoyl glycerol.

45. A pharmaceutical composition useful for promoting the brain uptake of a GABA- or GABA analogue-containing compound, comprising:

an effective amount of the compound of Claim 1, or a combination thereof; and

a pharmaceutically acceptable excipient.

46. The composition of Claim 45, wherein the excipient is suitable for the oral administration of the compound.

47. The composition of Claim 46, wherein said composition is in the form of dragees, tablets, syrups and ampules.

48. The composition of Claim 47, in unitary dosage form.

49. The composition of Claim 45, wherein the excipient is suitable for the rectal administration of the compound.

50. The composition of Claim 49, wherein said composition is in the form of a suppository.

51. The composition of Claim 50, in unitary dosage form.

52. The composition of Claim 45, wherein the excipient is suitable for the subcutaneous, intramuscular or intravenous administration of the composition.

53. The composition of Claim 52, in unitary dosage form.

54. The composition of Claim 45, wherein the excipient is suitable for the topical application of the compound.

55. The composition of Claim 54, wherein the composition is in the form of a pomade or a gel.

56. The composition of Claim 55, in unitary dosage form.

57. Use of the comopund of Claim 1 in the preparation of a medicament for promoting the crossing of the blood-brain barrier of a patient by said compound.

58. Use of the comopund of Claim 1 for the preparation of a medicament for treatment of abnormal GABA levels in the brain.

59. The medicament of Claim 58 for the treatment of epilepsy, Huntingdon's disease, manic depression, general dispression, schizophrenia and neuropsychiatric disorders.

60. Use of the compound of Claim 1 for the preparation of a medicament for regulating general locomotor activity.

61. Use of the comopund of Claim 1 for the preparation of a medicament useful for preventing or treating a condition associated with seizures.

62. A process of preparing the compound of Claim 1, comprising:

(a) reacting GABA or GABA analogue with 2-[(t-butoxycarbonyl)oximino]-2-phenylacetonitrile to obtain t-butoxycarbonyl-GABA or t-butoxycarbonyl-GABA analogue;

(b) dehydrating the t-butoxycarbonyl-GABA or t-butoxycarbonyl-GABA analogue in the presence of a dehydrating agent to obtain t-butoxycarbonyl-GABA anhydride or t-butoxycarbonyl-GABA analogue anhydride;

(c) esterifying the t-butoxycarbonyl GABA anhydride or t-butoxycarbonyl-GABA analogue anhydride with a compound of the formula:

$$AOH$$

to obtain a t-butoxycarbonyl-GABA-A or t-butoxycarbonyl-GABA analogue-A ester; and

(d) deamidifying the t-butoxycarbonyl-GABA or t-butoxycarbonyl-GABA analogue ester to obtain the deamidified GABA-A or GABA analogue-A ester.

63. The process of Claim 62, wherein the dehydrating agent of step (b), is dicyclohexylcarbodiimide.

64. The process of Claim 62 wherein the deamidification step (d) is carried out in the presence of trifluoroacetic acid.

65. A process for preparing the compound of Claim 34, comprising:

(a) protecting two of the OH groups contained in the glyceryl compound by forming a glyceryl acetonide of the formula:

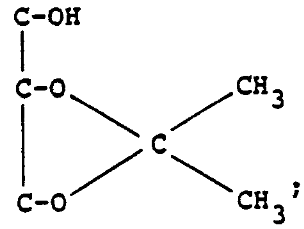

(b) esterifying the free OH group by reacting the glyceryl acetonide with an anhydride of the formula:

$$R_2O$$

wherein R is a radical of a carboxylic acid as defined above, in a dry organic solvent, in the presence of 4-dimethylaminopyridine, and whereby a monoester of the glyceryl acetonide is obtained;

(c) freeing the two previously protected OH groups by reacting the monoester of a diglyceryl acetonide with an acid to obtain a diglyceryl-R monoester;

(d) esterifying a t-butoxycarbonyl GABA anhydride or t-butoxycarbonyl GABA analogue anhydride with the diglyceryl-R monoester in a molar ratio of about 2:1 or greater, and in the presence of 4-dimethylaminopyridine in benzene, thereby obtaining a di-t-butoxycarbonyl-GABA-R-glyceryl ester or di-t-butoxycarbonyl-GABA-analogue-R-glyceryl ester; and

(e) deamidifying the di-t-butoxycarbonyl-GABA-R-glyceryl ester or di-t-butoxycarbonyl-GABA analogue-R-glyceryl ester, thereby obtaining the deamidified diGABA-R-glyceryl ester or di-GABA analogue-R-glyceryl ester.

66. The process of Claim 65, where the acid of step (c) is trifluoroacetic acid.

67. The process of Claim 65, wherein step (e) is replaced by the following steps:

(a) converting the thus obtained glyceryl ester to the corresponding glyceryl ester-trifluoroacetate salt in the presence of trifluoroacetic acid in an atmosphere of $N_2$ gas; and

(b) extracting the di-GABA or di-GABA analogue, R glyceryl triester-trifluoroacetate salt with a mixture of aqueous $NaHCO_3$ and an organic solvent, and whereby the free di-GABA or di-GABA analogue-R-glyceryl triester is obtained.

68. The process of Claim 65, wherein the molar ratio of the t-butoxycarbonyl GABA or GABA analogue anhydride and the diglyceryl-R monoester in step (d) is less than 1:1; and further comprising, after step (d), the following additional step:

(e) esterifying the t-butoxycarbonyl GABA or GABA analogue-R-monoglyceryl ester with a compound selected from:

$$R—COCl \text{ or } (RCO)_2O,$$

whereby a t-butoxycarbonyl GABA or GABA analogue, diR glyceryl ester is obtained.

69. The process of Claim 63, wherein the anhydride of step (b) is N-carbobenzoxy-GABA or GABA analogue anhydride and the product obtained thereof is an N-carbobenzoxy-GABA or -GABA analogue ester of the glyceryl acetonide; and further comprising the following steps thereafter;

(c) hydrogenating the thus obtained N-carbobenzoxy-GABA or GABA analogue ester of the glyceryl acetonide with hydrogen gas in the presence of Pd-C to obtain the diglyceryl ester of N-carbobenzoxy-GABA or -GABA analogue;

(d) reacting the thus obtained diglyceryl ester of N-carbobenzoxy-GABA or -GABA analogue with trifluoroacetic acid to obtain the trifluoroacetate salt of the diglyceryl ester of GABA or -GABA analogue; and

(e) forming an HCl addition salt of the diglyceryl ester of GABA or GABA analogue by reacting the product of step (d) with HCl.

70. The compound of Claim 1, wherein $R_2$, $R_3$ and $R_4$ are hydrogen, and $R_1$ is vinyl.

71. The compound of Claim 70 wherein A is glucosyl.

72. The compound of Claim 70 wherein A is cholesteryl.

73. The compound of Claim 1 which is gamma-vinyl GABA-di-linolenoyl-glyceryl triester.

74. The compound of Claim 1 which is di-(gamma-vinyl GABA)-linolenoyl-glyceryl triester.

75. The compound of Claim 1 wherein A represents the radical of dihydroxy acetone.

## Patentansprüche

1. Verbindung der Formel

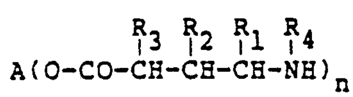

worin

$R_1$, $R_2$ und $R_3$ gleich oder verschieden sind und ausgewählt sind aus:
(a) Wasserstoff;
(b) niedere Alkylgruppen mit ein bis vier Kohlenstoffatomen;
(c) substituierten niederen Alkylgruppen mit ein bis vier Kohlenstoffatomen;
(d) niederen Alkenylgruppen mit ein bis vier Kohlenstoffatomen;
(e) substituierten niederen Alkenylgruppen mit ein bis vier Kohlenstoffatomen;
(f) niederen Alkylnylgruppen mit ein bis vier Kohlenstoffatomen;
(g) substituierten niederen Alkylnylgruppen mit ein bis vier Kohlenstoffatomen;
(h) Arylgruppen;
(i) substituierten Arylgruppen;
(j) Hydroxygruppen oder mit niederen Acyl- oder Aroylgruppen geschützte Hydroxygruppen;
(k) niederen Acylgruppen;
(l) Oxogruppen, wobei in diesem Falle am Kohlenstoffatom kein Wasserstoff ist;
(m) Aminogruppen;
(n) substituierten Aminogruppen;
(o) $R_1$ und $R_3$, die zusammen einen Kohlenstoffring bilden;
(p) $R_2$ und $R_3$ die zusammen einen Kohlenstoffring bilden;

$R_4$ Wasserstoff oder Acyl ist;

A einen Rest einer Verbindung mit wenigstens einer veresterbaren OH-Gruppe darstellt, die aus der aus Kohlenhydraten, gesättigten aliphatischen Alkoholen mit 4—40 Kohlenstoffatomen und mehr als einem OH-Substituenten, ungesättigten aliphatischen Alkoholen, zyklischen Alkoholen, aromatischen Alkoholen, Alkoholen mit wenigstens einem Heteroatom und Glycerylestern fettiger Säuren bestehenden Gruppe ausgewählt und in der Lage ist, die Blut-Hirn-Schranke zu überwinden;

n von 1 bis zu der gesamten Anzahl der in A enthaltenen veresterbaren OH-Gruppen variieren kann; oder

pharmazeutisch verträgliche Säureadditonssalz davon, wobei der Ester einen Gehirnpenetrationsindex von größer als etwa 2% hat.

2. Verbindung nach Anspruch 1, mit einem Gehirnpenetrationsindex von größer als etwa 3%.

3. Verbindung nach Anspruch 2, mit einem Gehirnpenetrationsindex von größer als etwa 25%.

4. Verbindung nach Anspruch 1, mit einem n-Octanol/Wasserverteilungskoeffizienten von größer als etwa 1.

5. Verbindung nach Anspruch 4 mit einem n-Octanol/Wasserverteilungskoeffizienten von größer als etwa 2.

6. Verbindung nach Anspruch 5 mit einem n-Octanol/Wasserverteilungskoeffizienten von größer als etwa 6.

7. Verbindung nach Anspruch 1, worin A den Rest eines Kohlenhydrats mit einer Kohlenstoffkette von 3 bis 20 Kohlenstoffatomen darstellt.

8. Verbindung nach Anspruch 7, worin die Kohlenstoffkette 3 bis 7 Kohlenstoffatome enthält.

9. Verbindung nach Anspruch 7, worin das Kohlenhydrat die allgemeine Formel

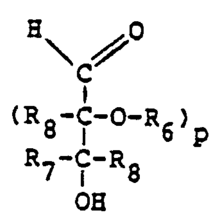

hat, worin

(1) p 1 bis 18 ist,

(2) $R_6$, $R_7$ und $R_8$ gleich oder verschieden sein können, und
(a) H;
(b) OH, mit der Ausnahme, daß $R_7$ und $R_8$ nicht OH sein können;
(c) verzweigtes oder unverzweigtes Amino der Formel $(CH_2)_nNH_2$, worin n 1 bis 15 ist;
(d) verzweigtes oder unverzweigtes Hydrazino der Formel $(CH_2)_nNHNH_2$, worin n wie oben definiert ist;
(e) Haloalkyl der Formel $(CH_2)_nCX_mH_{(3-m)}$, worin n wie oben definert ist und m 1 bis 3 ist;
(f) Alkyl mit 1 bis 15 Kohlenstoffatomen;
(g) Alkenyl mit 1 bis 15 Kohlenstoffatomen;
(h) Alkynyl mit 1 bis 15 Kohlenstoffatomen; oder
(i) $(Ch_2)_nOPO_3H_2$, worin n wie oben definiert ist, darstellen und zusätzlich

(3) $R_7$ und $R_8$ gleich oder verschieden sein können und Haloalkyl der Formel $(CX_mH_{(3-)}$, worin m und X wie oben definiert sind, darstellen; und

(4) $R_6$ Acetyl darstellt.

10. Verbindung nach Anspruch 5, worin das Kohlenhydrat aus der aus D-Glucose; D-Glycerinaldehyd; D-Erythrose; D-Threose; D-Ribose; D-Arabinose; D-Xylose; D-Allose; D-Mannose; D-Gulose; D-Idose; D-Galactose; D-Talose; Dihydroxyaceton; D-Erythrulose; D-Ribulose; D-Xylulose; D-Psikose; D-Fructose; D-Sorbose; ihre O-Acetylderivate; ihre O-Nethylderivate; ihre Desoxy-D-Derivate; ihre Amino-D-Derivate; und ihre Säure-D-Derivate bestehenden Gruppe ausgewählt ist.

11. Verbindung nach Anspruch 1, worin A den Rest eines verzweigten oder unverzweigten gesättigten aliphatischen Alkohols mit mehr als einem OH-Substituenten und einer Kohlenstoffkette von 4 bis 40 Kohlenstoffatomen darstellt.

12. Verbindung nach Anspruch 1, worin A einen Rest eines verzweigten oder unverzweigten aliphatischen Alkohols mit wenigstens einer ungesättigten Stelle darstellt.

13. Verbindung nach Anspruch 12, worin die Kohlenstoffkette 4 bis 40 Kohlenstoffatome enthält.

14. Verbindung nach Anspruch 13, worin die Kohlenstoffkette 4 bis 30 Kohlenstoffatome enthält.

15. Verbindung nach Anspruch 14, worin die Kohlenstoffkette 8 bis 20 Kohlenstoffatome enthält.

16. Verbindung nach Anspruch 12, worin der Alkohol ein Alkenyl ist.

17. Verbindung nach Anspruch 13, worin der Alkohol ein Alkynyl ist.

18. Verbindung nach Anspruch 5, worin der Alkohol die Formel

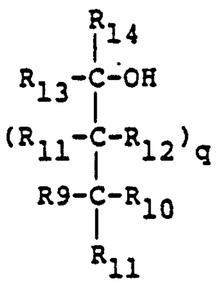

hat, worin:

(1) q bis 38 ist;

(2) $R_9$, $R_{10}$, $R_{11}$, $R_{12}$, $R_{13}$ und $R_{14}$ gleich oder verschieden sein können und

(a) H, mit der Bedingung, daß wenigstens einer von H verschieden ist;

(b) OH, mit der Ausnahme, daß $R_{13}$ und $R_{14}$ nicht OH sein können;

(c) verzweigtes oder unverzweigtes Amin der Formel $(CH_2)_nNH_2$, worin n 1 bis 15 ist;

(d) verzweigtes oder unverzweigtes Alkyl mit 1 bis 15 Kohlenstoffatomen;

(e) verzweigtes oder unverzweigtes Hydrazino der Formel $(Ch_2)_nNH—NH_2$, worin n wie oben definiert ist;

(f) verzweigtes oder unverzweigtes Haloalkyl der Formel $(CH_2)_nCX_mH_{(3-m)}$, worin X für F, Cl, Br oder I steht, n wie oben definiert ist und m 1 bis 3 ist;

(g) verzweigtes oder unverzweigtes Alkenyl mit 1 bis 15 Kohlenstoffatomen;

(h) verzweigtes oder unverzweigtes Alkynyl mit 1 bis 15 Kohlenstoffatomen;

(i) Acetyl;

(j) ihre O-Acetylderivate; und

(k) ihre O-Methylderivate darstellen.

19. Verbindung nach Anspruch 18, worin der Alkohol einen oder mehrere Kohlenstoffringe aufweist und $R_{10}$ und $R_{12}$ eine zyklische Struktur aufweisen kann, die einen oder mehrere Kohlenstoffringe enthält.

20. Verbindung nach Anspruch 19, worin ein oder mehrere der Kohlenstoffringe im zyklischen Alkohol aromatisch sind.

21. Verbindung nach Anspruch 20, worin der aromatische Alkohol ein Polyalkohol ist.

22. Verbindung nach Anspruch 1, worin A einen Rest eines verzweigten oder unverzweigten, gesättigten oder ungesättigten zyklischen Alkohols mit maximal 18 Ringkohlenstoffatomen darstellt.

23. Verbindung nach Anspruch 22 mit 5 bis 14 Ringkohlenstoffatomen.

24. Verbindung nach Anspruch 22 mit 6 bis 10 Ringkohlenstoffatomen.

25. Verbindung nach Anspruch 22, worin der zyklische Alkohol aus der aus Cyclopentanol; Cyclohexanol, Cycloheptanol; Cyclooctanol; Cyclodecanol; Cyclododecanol; Cyclotetradecanol; Cyclohexadecanol; Cyclooctadecanol; Inositol; ihren Derivaten mit mehrfachen Hydroxylsubstituenten; ihren O-Acetylderivaten; ihren O-Methylderivaten; ihren Aminderivaten; und ihren halogenierten Derivaten bestehenden Gruppe ausgewählt ist.

26. Verbindung nach Anspruch 22, worin der zyklische Alkohol einen oder mehrere aromatische Ringe aufweisen kann.

27. Verbindung nach Anspruch 1, worin A einen Rest eines verzweigten oder unverzweigten Alkohols mit 4 bis 40 Kohlenstoffatomen darstellt, der ein oder mehrere Heteroatome besitzt.

28. Verbindung nach Anspruch 27, worin A 1 bis 3 Sauerstoff-, Stickstoff- oder Schwefelatome oder eine Kombination davon enthält.

29. Verbindung nach Anspruch 28, worin A aus der Gruppe von Alkyl-, Alkenyl- und Alkynylalkoholen mit einer Kohlenstoffkette von 4 bis 40 Kohlenstoffatomen ausgewählt ist.

30. Verbindung nach Anspruch 28, worin der Alkohol ein zyklischer Alkohol mit 5 bis 18 Ringkohlenstoffatomen ist.

31. Verbindung nach Anspruch 30 mit 6 bis 10 Ringkohlenstoffatomen.

32. Verbindung nach Anspruch 27, worin der Alkohol einen oder mehrere aromatische Ringe aufweist.

33. Verbindung nach Anspruch 27, worin der Heteroalkohol aus der aus Ethanolamin, Chlorin, Serin, O-Aminoacylglycerol, ihrer Alkylderivate und ihrer O-Acylderivate bestehenden Gruppe ausgewählt ist.

34. Verbindung nach Anspruch 1, worin A den Rest eines mit bis zu zwei Carboxylsäureresten, die gleich oder verschieden sein können, veresterten Glycerols darstellt.

35. Verbindung nach Anspruch 34, worin die Carboxylsäuren verzweigte oder unverzweigte aliphatische Säuren sind.

36. Verbindung nach Anspruch 35 mit 10 bis 35 Kohlenstoffatomen in der Kette.

37. Verbindung nach Anspruch 35, worin die Carboxylsäuren eine gleiche Anzahl von Kohlenstoffatomen aufweisen.

38. Verbindung nach Anspruch 34, worin die Carboxylsäuren wenigstens eine ungesättige Stelle aufweisen.

39. Verbindung nach Anspruch 38, worin sich die Carboxylsäuren aus Alkenyl- oder Alkynylresten mit mehr als einer ungesättigten Stelle ableiten.

40. Verbindung nach Anspruch 34, worin der Glycerolrest mit einer Fettsäure und einer GABA oder einem GABA-Analogon verestert ist.

41. Verbindung nach Anspruch 40, worin der Glycerolrest mit einer Fettsäure und zwei GABAs oder zwei Analoga der GABA verestert ist.

42. Verbindung nach Anspruch 40, worin der Glycerolrest mit zwei Fettsäuren und einer GABA oder einem GABA-Analogon verestert ist.

43. Verbindung nach Anspruch 35, worin die Carboxylsäuren aus der aus Laurinsäure, Myristinsäure, Palmitinsäure, Stearinsäure, Arachinsäure, Palmitoleinsäure, Oleinsäure, Linolsäure, Linolensäure, Arachidonsäure, Cerebronsäure, Carbiolypin, ihren O-Acetylderivaten, ihren O-Methylderivaten, und ihren Aminoderivaten bestehenden Gruppe ausgewählt sind.

44. Verbindung nach Anspruch 1, worin A der Rest ist, der sich von einer Verbindung aus der aus Inositol, Cholesterol, Dexamethason, Dilinolenoylglycerol und Linolenoylglycerol bestehenden Gruppe ableitet.

45. Pharmazeutische Zubereitung zur Förderung der Aufnahme einer GABA oder eines GABA-Analogons enthaltenden Verbindung, gekennzeichnet durch eine wirksame Menge einer Verbindung des Anspruchs 1 oder eine Kombination davon; und einem pharmazeutisch akzeptierbaren Arzneimittelträger.

46. Zubereitung nach Anspruch 45, worin der Arzneimittelträger zur oralen Verabreichung der Verbindung geeignet ist.

47. Zubereitung nach Anspruch 46, worin diese Zubereitung in Form von Dragees, Tabletten, Sirupen und Ampullen vorliegt.

48. Zubereitung nach Anspruch 47 in Einheitsdosierungsform.

49. Zubereitung nach Anspruch 45, worin der Arzneimittelträger zur rectalen Verabreichung der Verbindung geeignet ist.

50. Zubereitung nach Anspruch 49, worin diese Zubereitung in Form von Suppositorien vorliegt.

51. Zubereitung nach Anspruch 50 in Einheitsdosierungsform.

52. Zubereitung nach Anspruch 45, worin der Arzneimittelträger zur subcutanen, intramusculären oder intravenösen Verabreichung der zubereitung geeignet ist.

53. Zubereitung nach Anspruch 52 in Einheitsdosierungsform.

54. Zubereitung nach Anspruch 45, worin die Aufnahmeform zur topischen Applikation der Verbindung geeignet ist.

55. Zubereitung nach Anspruch 54, worin die Zubereitung in Form einer Pomade oder eines Gels vorliegt.

56. Zubereitung nach Anspruch 55 in Einheitsdosierungsform.

57. Verwendung der Verbindung nach Anspruch 1 zur Herstellung eines Medikaments, das die Überwindung der Blut-Hirn-Schranke eines Patienten durch diese Verbindung unterstützt.

58. Verwendung der Verbindung nach Anspruch 1 zur Herstellung eines Medikaments zur Behandlung anormaler GABA-Spiegel im Gehirn.

59. Medikament nach Anspruch 58 zur Behandlung von Epilepsie, Huntingdon's-Krankheit, manischer Depression, allgemeiner Depression, Schizophrenie und neuropsychiatrischer Störungen.

60. Verwendung der Verbindung nach Anspruch 1 zur Herstellung eines Medikaments zur Regulierung allgemeiner locomotorischer Aktivität.

61. Verwendung der Verbindung nach Anspruch 1 zur Herstellung eines Medikamentes, das nützlich ist zur Vorbeugung oder Behandlung von Krankheitszuständen, die mit Anfällen verbunden sind.

62. Verfahren zur Herstellung der Verbindung nach Anspruch 1, gekennzeichnet durch

(a) Umsetzung von GABA oder einem GABA-Analogon mit 2-[(t-Butoxycarbonyl)oximino]-2-phenylacetonitril, um t-Butoxycarbonyl-GABA oder ein t-Butoxycarbonyl-GABA-Analogon zu erhalten;

(b) Dehydratisierung der t-Butoxycarbonyl-GABA oder des t-Butoxycarbonyl-GABA-Analogons in Gegenwart eines Dehydratisierungsmittels, um t-Butoxycarbonyl-GABA-Anhydrid oder ein t-Butoxycarbonyl-GABA-Analogon-anhydrid zu erhalten;

(c) Veresterung des t-Butoxycarbonyl-GABA-Anhydride oder des t-Butoxycarbonyl-GABA-Analogon-Anhydrids mit einer Verbindung der Formel

AOH

um einen t-Butoxycarbonyl-GABA—A- oder einen t-Butoxycarbonyl-GABA-Analogon-A-Ester zu erhalten; und

(d) Desamidieren des t-Butoxycarbonyl-GABA- oder t-Butoxycarbonyl-GABA-Analogon-Ester, um einen desamidierten GABA—A- oder einer GABA-Analogon-A-Ester zu erhalten.

63. Verfahren nach Anspruch 62, worin das Dehydratisierungsmittel aus Stufe (b) Dicyclohexylcarbodiimid ist.

64. Verfahren nach Anspruch 62, worin die Desamidierungsstufe (d) in Gegenwart von Trifluoressigsäure durchgeführt wird.

65. Verfahren zur Herstellung der Verbindung nach Anspruch 34, gekennzeichnet durch

(a) Schützen von zwei der OH-Gruppen, die in der Glycerylverbindung enthalten sind, durch Bildung eines Glycerylacetonids der Formel:

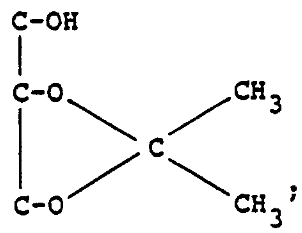

(b) Veresterung der freien OH-Gruppe durch Umsetzung des Glycerylacetonids mit einem Anhydrid der Formel

$R_2O$

worin R ein Rest einer wie oben definierten Carboxylsäure ist, in einem trockenen organischen Lösungsmittel in Gegenwart von 4-Dimethylaminopyridin, wobei ein Monoester des Glycerylacetonids erhalten wird,

(c) Freilegung der beiden zuvor geschützten OH-Gruppen durch Umsetzung des Monoesters von Diglycerylacetonid mit einer Säure, um einen Diglyceryl-R-monoester zu erhalten;

(d) Veresterung eines t-Butoxycarbonyl-GABA-Anhydrids oder t-butoxycarbonyl-GABA-Analogon-Anhydrids mit dem Diglyceryl-R-monoester in einem Molverhältnis von etwa 2:1 oder größer in Gegenwart von 4-Dimethylaminopyridin in Benzol, um dabei einen Di-t-butoxycarbonyl-GABA-R-glycerylester oder einen Di-t-butoxycarbonyl-GABA-Analogon-R-glycerylester zu erhalten; und

(e) Desamidierung des Di-t-butoxycarbonyl-GABA-R-glycerylesters oder des Di-t-butoxycarbonyl-GABA-Analogon-R-glycerylesters, wobei der desamidierte DiGABA-R-glycerylester oder Di-GABA-Analogon-R-glycerylester erhalten wird.

66. Verfahren nach Anspruch 65, worin die Säure der Stufe

(c) Trifluoroessigsäure ist.

67. Verfahren nach Anspruch 65, worin Stufe (e) durch die folgenden Stufen ersetzt ist:

(a) Überführung des so erhaltenen Glycerylesters in das korrespondiere Glycerylester-trifluoroacetatsalz in Gegenwart von Trifluoressigsäure in einer $N_2$-Gasatmosphäre; und

(b) Extraktion des Di-GABA- oder des Di-GABA-Analogons-R-Glyceryltriestertrifluoroacetatsalzes mit einer Mischung aus wässrigem $NaHCO_2$ und einem organischen Lösungsmittel, wobei freier Di-GABA- oder Di-GABA-Analogon-R-Glyceryltri- ester erhalten wird.

68. Verfahren nach Anspruch 65, worin das Molverhältnis von t-Butoxycarbonyl-GABA- oder GABA-Analogon-Anhydrid und dem Diglyceryl-R-monoester in Stufe (d) geringer als 1:1 ist; und weiterhin nach Stufe (d) die folgende zusätzliche Stufe umfaßt:

(e) Veresterung des t-Butoxycarbonyl-GABA- oder GABA-Analogen-R-monoglycerylesters mit einer Verbindung ausgewählt aus

R—COCl oder $(RCO)_2O$,

wobei ein t-Butoxycarbonyl-GABA- oder GABA-Anologon-di-R-glycerylester erhalten wird.

69. Verfahren nach Anspruch 63, worin das Anhydrid aus Stufe

(b) N-Carbobenzoxy-GABA- oder —GABA-Analogon-Anhydrid ist und das Produkt davon ein N-carbobenzoxy-GABA- oder —GBAB-Analogonester des Glycerylacetonids ist; und weiterhin folgende sich anschließende Stufen umfaßt;

(c) Hydrogenierung des so erhaltenen N-Carbobenzoxy-GABA- oder —GABA-Analogonester des Glycerylacetonids mit Wasserstoffgas in Gegenwart von Pd-C, um einen Diglycerylester des N-carbobenzoxy-GABA oder —GABA-Analogons zu erhalten;

(d) Umsetzung des so erhaltenen Diglycerylesters der N-Carbobenzoxy-GABA oder des —GABA-Analogons mit Trifluoressigsäure, um das Trifluoroessigsäuresalz des Diglycerylesters der GABA oder des GABA-Analogons zu erhalten; und

(e) Bildung eines HCl Additionssalzes des Diglycerylesters der GABA oder des GABA-Analogons durch umsetzung des Produktes der Stufe (d) mit HCl.

70. Verbindung nach Anspruch 1, worin $R_2$, $R_3$ und $R_4$ Wasserstoff sind und $R_1$ Vinyl ist.

71. Verbindung nach Anspruch 70, worin A Glucosyl ist.

72. Verbindung nach Anspruch 70, worin A Cholesteryl ist.

73. Verbindung nach Anspruch 1, welche Gamma-Vinyl-GABA-di-linolenoyl-glyceryltriester ist.

74. Verbindung nach Anspruch 1, welche Di-(Gamma-vinyl-GABA)-linolenoyl-glyceryltriester ist.

75. Verbindung nach Anspruch 1, worin A einen Dihydroxyacetonrest darstellt.

**Revendications**

1. Composé de formule:

$$A(O-CO-\overset{R_3}{\overset{|}{C}H}-\overset{R_2}{\overset{|}{C}H}-\overset{R_1}{\overset{|}{C}H}-\overset{R_4}{\overset{|}{N}H})_n$$

dans laquelle:

$R_1$, $R_2$, et $R_3$ sont semblables ou différents et sont choisis parmi:

(a) l'hydrogène,

(b) les groupes alkyle inférieur ayant de un à quatre atomes de carbone;

(c) les groupes alkyle inférieur substitué ayant de un à quatre atomes de carbone;

(d) les groupes alkényle inférieur ayant de un à quatre atomes de carbone;

(e) les groupes alkényle inférieur substitué ayant de un à quatre atomes de carbone;

(f) les groupes alkynyle inférieur ayant de un à quatre atomes de carbones;

(g) les groupes alkynyle inférieur substitué ayant de un à quatre atomes de carbone;

(h) les groupes aryle;

(i) les groupes aryle substitué;

(j) les groupes hydroxy ou les groupes hydroxy protégés par des groupes acyle inférieur ou des groupes aroyle;

(k) les groupes acyle inférieur;

(l) les groupes oxo, auquel cas l'hydrogène n'est pas présent sur l'atome de carbone;

(m) les groupes amino;

(n) les groupes amino substitué;

(o) $R_1$ et $R_2$ ensemble formant un noyau carbocyclique;

(p) $R_2$ et $R_3$ formant ensemble un noyau carbocyclique;

$R_4$ est l'hydrogène ou un groupe acyle;

A représente le radical d'un composé ayant au moins un groupe OH estérifiable, choisi dans le groupe comprenant les carbohydrates, les alcools aliphatiques saturés ayant de 4 à 40 atomes de carbone et plus d'un substituant OH, les alcools, aliphatiques insaturés, les alcools cycliques, les alcools aromatiques, les alcools contenant au moins un hétéroatome et des esters glycéryliques d'acides gras, et étant capables de franchir la barrière hémato-encéphalique d'un animal;

n peut varier de 1 au nombre total des groupes OH estérifiables contenus dans A;

ou un sel d'addition de celui-ci pharmaceutiquement acceptable,

ledit ester ayant un indice de pénétration dans le cerveau plus grand qu'environ 2%

2. Le composé de la revendication 1, ayant un indice de pénétration dans le cerveau plus grand qu'environ 3%.

3. Le composé de la revendication 2 ayant un indice de pénétration dans le cerveau plus grand qu'environ 25%.

4. Le composé de la revendication 1 ayant un coefficient de partition n-octanol/eau plus grand qu'environ 1.

5. Le composé de la revendication 4 ayant un coefficient de partition n-octanol/eau plus grand qu'environ 2.

6. Le composé de la revendication 5, ayant un coefficient de partition n-octanol/eau plus grand qu'environ 6.

7. Le composé de la revendication 1, dans lequel A représente un radical de carbohydrate ayant une chaîne carboné de 3 à 20 atomes de carbone.

8. Le composé de la revendication 7, dans lequel la chaîne carbonée contient de 3 à 7 atomes de carbone.

9. Le composé de la revendication 7, dans lequel le carbohydrate a formule générale:

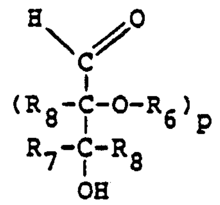

dans laquelle

(1) p vaut de 1 à 18,

(2) $R_6$, $R_7$ et $R_8$ peuvent être semblables ou différents et représentent:

(a) H;

(b) OH, excepté que $R_7$ et $R_8$ ne peuvent pas être OH;

(c) un groupe amino ramifié ou linéaire de formule $(CH_2)_n\ NH_2$, dans laquelle n vaut de 1 à 15;

(d) un groupe hydrazino ramifié ou linéaire de formule $(CH_2)_nNHNH_2$, dans laquelle n est défini comme ci-dessus;

(e) un haloalkyle de formule $(CH_2)_nCX_mH_{(3-m)}$, dans laquelle n est défini comme ci-dessus et m vaut de 1 à 3;

(f) un groupe alkyle ayant de 1 à 15 atomes de carbone;

(g) un groupe alkényle ayant de 1 à 15 atomes de carbone;

(h) un groupe alkynyle ayant de 1 à 15 atomes de carbone; ou

(i) $(CH_2)_nOPO_3H_2$, dans lequel n est défini comme ci-dessus; et en outre

(3) $R_7$ et $R_8$ peuvent être semblables ou différents et représentent un groupe haloalkyle de formule $(CX_mH_{(3-m)}$ dans laquel m et X sont définis comme ci-dessus; et

(4) $R_6$ représente un groupe acétyle.

10. Le composé de la revendication 5 dans lequel 1c carbohydrate est choisi dans le groupe comprenant le D-glucose; le D-glycéraldéhyde; le D-érythrose; le D-thréose, le D-ribose, le D-arabinose, le D-xylose; le D-allose; le D-mannose; le D-gulose, le D-idose, le D-galactose, le D-talose, La D-dihydroxy-acétone, le D-érythrulose, le D-ribulose, le D-xylulose, le D-psikose, le D-fructose, D-sorbose; leurs dérivés O-acétyl; leurs dérivés O-méthyl; leurs dérivés-D-déoxy; leurs dérivés-D-amino et leurs dérivés-D-acide.

11. Le composé de la revendication 1, dans lequel A représente le radical d'un alcool aliphatique saturé linéaire ou ramifié ayant plus d'un substituant OH et une chaîne carbonée de 4 à 40 atomes de carbone.

12. Le composé de la revendication 1, dans lequel A représente la radical d'un alcool aliphatique ramifié ou linéaire, ayant au moins un site d'insaturation.

13. Le composé de la revendication 12, dans lequel le chaîne carbonée contient de 4 à 40 atomes de carbone.

14. Le composé de la revendication 13, dans lequel la chaîne carbonée contient de 4 à 30 atomes de carbone.

15. Le composé de la revendication 14, dans lequel la chaîne carbonée contient de 8 à 20 atomes de carbone.

16. Le composé de la revendication 12, dans lequel l'alcool est alkénylique.

17. Le composé de la revendication 13, dans lequel l'alcool est alkynylique.

18. Le composé de la revendication 5, dans lequel l'alcool a la formule

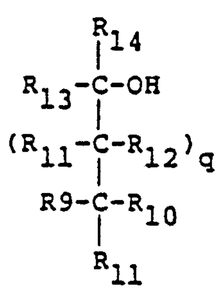

dans laquelle:

(1) q est égal à 1 à 38;

(2) $R_9$, $R_{10}$, $R_{11}$, $R_{10}$, $R_{12}$, $R_{13}$ et $R_{14}$ peuvent ètre semblables ou différents et représentent:

(a) H, à la condition qu'au moins un de ceux-ci soit différent de H;

(b) OH, excepté que $R_{13}$ et $R_{14}$ ne peuvent pas être OH.

(c) une amine ramifiée ou linéaire de formule $(CH_2)_nNH_2$, dans laquelle n vaut de 1 à 15.

(d) un alkyle ramifié ou linéaire ayant de 1 à 15 atomes de carbone;

(e) un groupe hydrazino ramifié ou linéaire de formule $(CH_2)_nNH—NH_2$, dans lequel n est défini comme ci-dessus;

(f) un haloalkyle ramifié ou linéaire de formule $(CH_2)_nCX_mH_{(3-m)}$, dans laquelle X représente F, Cl, Br, ou I, n est défini comme ci-dessus, et m vaut de 1 à 3;

(g) un alkényle ramifié ou linéaire de 1 à 15 atomes de carbone;
(h) un groupe alkynyle ramifié ou linéaire de 1 à 15 atomes de carbone;
(i) le groupe acétyle;
(j) leurs dérivés O-acétyl; et
(k) leurs dérivés O-méthyl.

19. Le composé de la revendication 18, dans lequel l'alcool a au moins un noyau carboné, et $R_{10}$ et $R_{12}$ peuvent être une structure cyclique contenant au moins un noyau carboné.

20. Le composé de la revendication 19, dans lequel au moins un des noyuau carboné de l'alcool cyclique est aromatique.

21. Le composé de la revendication 20, dans lequel l'alcool aromatique est un polyalcool.

22. Le composé de la revendication 1, dans lequel A représente un radical d'un alcool cyclique saturé ou insaturé, ramifié ou linéaire ayant un maximum de 18 atomes de carbone cycliques.

23. Le composé de la revendication 22 ayant de 5 à 14 atomes cyliques de carbone.

24. Le composé de la revendication 22 ayant de 6 à 10 atomes cycliques de carbone.

25. Le composé de la revendication 22 dans lequel l'alcool cyclique est choisi dans le groupe comprenant le cyclopentanol, le cyclohexanol, le cycloheptanol, le cyclooctanol, le cyclodécanol, le cyclododécanol, le cyclotétradécanol, le cyclohexadécanol, le cyclooctadécanol, l'inositol, leurs dérivés ayant des substituants hydroxyl multiples; leurs dérivés O-acétyl; leurs dérivés O-méthyl; leurs dérivés aminés et leurs dérivés halogénés.

26. Le composé de la revendication 22, dans lequel l'alcool cyclique peut comporter un noyau aromatique ou plus.

27. Le composé de la revendication 1, dans lequel A représente le radical d'un alcool ramifié ou linéaire ayant de 4 à 40 atomes de carbone et ayant au moins un hétéroatome.

28. Le composé de la revendication 27, dans lequel A contient de 1 à 3 atomes d'oxygène, d'azote ou de soufre, ou une combinaison de ceux-ci.

29. Le composé de la revendication 28, dans lequel A est choisi dans le groupe comprenant les alcools alkyliques, alkényliques et alkynyliques ayant une chaîne carbonée de 4 à 40 atomes de carbone.

30. Le composé de la revendication 38, dans lequel l'alcool est un alcool cyclique de 5 à 18 atomes de carbonecycliques.

31. Le composé de la revendication 30, ayant de 6 à 10 atomes de carbonecyliques.

32. Le composé de la revendication 27, dans lequel l'alcool a au moins un noyau aromatique.

33. Le composé de la revendication 27, dans lequel l'hétéro alcool est choisi dans le groupe comprenant l'éthanolamine, la choline, la sérine, l'O-aminoacylglycérol, leurs dérivés alkylés, et leurs dérivés O-acyl.

34. Le composé de la revendication 1, dans lequel A représente le radical d'un glycérol estérifié avec jusqu'à deux radicaux d'acides carboxyliques pouvant être semblables ou différents.

35. Le composé de la revendication 34, dans lequel les acides carboxyliques sont des acides aliphatiques ramifiés ou linéaires.

36. Le composé de la revendication 35 ayant de 10 à 30 atomes de carbone dans la chaîne.

37. Le composé de la revendication 35, dans lequel les acides carboxyliques ont un nombre paire d'atomes de carbone.

38. Le composé de la revendication 34, dans lequel les acides carboxyliques contiennent au moins un site d'insaturation.

39. Le composé de la revendication 38, dans lequel les acides carboxyliques dérivent d'un radical alkényle ou alkynyle ayant plus d'un site d'insaturation.

40. Le composé de la revendication 34, dans lequel le radical glycérol est estérifié par un acide gras et un GABA ou un analoque du GABA.

41. Le composé de la revendication 40, dans lequel le radical glycérol est estérifié par un acide gras et deux GABAs ou deux analogues du GABA.

42. Le composé de la revendication 40, dans lequel le radical glycérol est estérifié par deux acides gras et un GABA ou un analogue du GABA.

43. Le composé de la revendication 35, dans lequel les acides carboxyliques sont choisis parmi un groupe comprenant l'acide laurique, l'acide myristique, l'acide palmitique, l'acide stéarique, l'acide arachidique, l'acide palmitoléique, l'acide oléique, l'acide linoléique, l'acide linolénique, l'acide arachidonique, l'acide cérébronique, la cardiolypine, leurs dérivés O-acétyl, leurs dérivés O-méthyl et leurs dérivés aminés.

44. Le composé de la revendication 1, dans lequel A est le radical dérivé d'un composé choisi dans le groupe comprenant l'inositol, le cholestérol, la dexaméthasone, le dilinolénoyl glycérol, et le linolénoyl glycérol.

45. Une composition pharmaceutique utile pour activer la capture par le cerveau d'un composé comportant du GABA ou un analogue du GABA, comprenant:
une quantité efficace du composé de la revendication 1, ou une combinaison de celui-ci; et
un excipient pharmaceutiquement acceptable.

46. La composition de la revendication 45, dans lequelle l'excipient convient à l'administration orale du composé.

47. La composition de la revendication 46, dans laquelle ladite composition est sous forme de dragées, de comprimés, de sirops et d'ampoules.

48. La composition de la revendication 47, sous forme de dosage unitaire.

49. La composition de la revendication 45, dans laquelle l'excipient convient à l'administation rectale du composé.

50. La composition de la revendication 49, dans laquelle ladite composition est sous la forme de suppositoire.

51. La composition de la revendication 50, sous forme de dosage unitaire.

52. La composition de la revendication 45, dans laquelle l'excipient convient à l'administration sous-cutanée, intramusculaire ou intraveineuse de la composition.

53. La composition de la revendication 52, sous forme de dosage unitaire.

54. La composition de la revendication 45, dans lequelle l'excipient convient à l'application topique du composé.

55. La composition de la revendication 54, dans lequelle la composition est sous la forme d'une pommade ou d'un gel.

56. La composition de la revendication 55, sous forme de dosage unitaire.

57. Utilisation d'une composé de la revendication 1, pour la préparation d'un médicament pour activer le passage de la barrière hématoencéphalique d'un patient par ledit composé.

58. Utilisation d'une composé de la revendication 1, pour la préparation d'un médicament pour le traitement de taux de GABA anormaux dans le cerveau.

59. Le médicament de la revendication 58 pour le traitement de l'épilepsie, de la maladie de Huntingdon, de la dépression maniaco-dépressive, de la dépression générale, de la schizophrénie et des désordres neuropsychiatriques.

60. Utilisation d'une composé de la revendication 1, pour la préparation d'un médicament en vue de la régulation de l'activité locomotrice générale.

61. Utilisation d'une composé de la revendication 1, pour la préparation d'un médicament utile pour la prévention ou le traitement d'un état associé avec des convulsions.

62. Procédé de préparation du composé de la revendication 1, où:

(a) on fait réagtir du GABA ou un analogue du GABA avec le 2-[(t-butoxycarbonyl-oximino]-2-phényl-acétonitrile pour obtenir le t-butoxycarbonyl-GABA ou un analoque du t-butoxylcarbonyl-analogue du GABA;

(b) on déshydrate le t-butoxycarbonyl-GABA ou le t-butoxycarbonyl-analogue du GABA en présence d'un agent de déshydratation pour obtenir l'anhydride du t-butoxycarbonyl-GABA ou l'anhydride du t-butoxycarbonyl-analogue du GABA;

(c) on estérific l'anhydride du t-butoxycarbonyl-GABA ou l'anhydride du t-butoxycarbonyl-analoque du GABA avec un composé de formule;

AOH

afin d'obtenir un ester t-butoxycarbonyl-GABA—A ou t-butoxycarbonyl-analogue du GABA; et

(d) on déamidifie l'ester du t-butoxycarbonyl-GABA ou du t-butoxycarbonyl-analogue du GABA pour obtenir l'ester déamidifié GABA—A ou de l'analogue du GABA—A.

63. Le procédé de la revendiction 62 dans lequel l'agent déshydratant de l'étape (b), est le dicyclohexylcarbodiimide.

64. La procédé de la revendication 62 dans lequel l'étape de déamidification (d) est effectuée en présence d'acide trifluoroacétique.

65. La procédé pour la préparation du composé de la revendication 34, où:

(a) on protège deux des groupes OH contenus dans le composé glycéryl en formant un acétonide de glycéryle de formule:

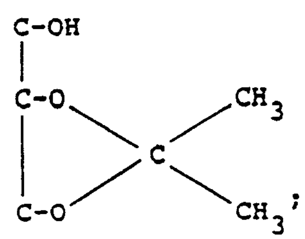

(b) on estérifie le groupe OH libre en faisant réagir l'acétonide de glycéryle avec un anhydride de formule:

$R_2O$

dans laquelle R est un radical d'un acide carboxylique comme défini ci-dessus, dans un solvant organique sec, en présence de 4-diméthylaminopyridine, et de façon à obtenir un monoester de l'acétonide de glycéryle;

(c) on libère les deux groupes OH protégés auparavant en faisant réagir le monoester de l'acétonide de diglycéryl avec un acide pour obtenir un monoester de diglycéryl-R;

(d) on estérifie un anhydride de t-butoxycarbonyl-GABA ou un anhydride de t-butoxycarbonyl-analogue du GABA avec le monoester diglycéryl-R dans un rapport molaire d'environ 2/1 ou plus, et en présence de 4-dimethylaminopyridine dans le benzène, pour obtenir un ester di-t-butoxycarbonyl-GABA—R-glycéryl ou di-t-butoxycarbonyl-analogue du GABA—R-glycéryl; et

(e) on deamidifie l'ester di-t-butoxycarbonyl-GABA R-glycéryl ou l'ester di-t-butoxycarbonyl-analogue du GABA—R-glycéryl, pour obtenir l'ester déamidifié di-GABA—R-glycéryl ou di-analogue du GABA- R-glycéryl.

66. Le procédé de la revendication 65, où l'acide de l'étape (c) est l'acide trifluoroacétique.

67. Le procédé de la revendication 65 dans lequel l'étape (e) est remplacée par les étapes suivantes:

(a) on converti l'ester glycéryl ainsi obtenu en sel correspondant trifluoroacétate du glyceryl ester, en présence d'acide trifluoroacétique dans une atmosphère de gaz $N_2$; et

(b) on extrait le sel trifluoroacétate du triester di-GABA—R-glycéryl ou du triester di-analogue du GABA R-glycéryl avec un mélange de $NaHCO_3$ aqueux et d'un solvant organique pour obtenir le triester libre di-GABA—R-glycéryl ou di-analogue du GABA—R-glycéryl.

68. Le procédé de la revendication 65, dans lequel le rapport molaire de l'anhydride du t-butoxycarbonyl-GABA ou de l'analogue du GABA et du monoester diglycéryl-R dans l'étape (d) est inférieur à 1/1; et qui comprend en outre, après l'étape (d) l'étape additionnelle suivante:

(e) on estérifie l'ester du t-butoxycarbonyl-GABA—R-monoglycéryl ou de l'analogue du GABA—R-monoglycéryl avec un composé choisi entre:

$$R—COCl \text{ ou } (RCO)_2O,$$

pour obtenir un ester t-butoxycarbonyl-GABA-di-R-glycéryl ou un ester analogue du GABA-di-R-glycéryl.

69. Le procédé de la revendication 63 dans lequel l'anhydride de l'étape (b) est l'anhydride du N-carbobenzoxy-GABA ou l'anhydride de N-carbobenzoxy analogue du GABA et le produit, obtenu ainsi, est un ester du N-carbobenzoxy-GABA ou du N-carbobenzoxy-analogue du GABA de l'acétonide de glycéryle; et en outre comprend les étapes suivantes:

(c) on hydrogène l'ester N-carbobenzoxy-GABA ou analogue du GABA de l'acétonide de glycéryle ainsi obtenu avec de l'hydrogène gazeux en présence de Pd-C pour obtenir l'ester diglycéryl du N-carbobenzoxy-GABA ou du N-carbobenzoxy-analogue du GABA;

(d) on fait réagir l'ester diglycérique de N-carbobenzoxy-GABA ou N-carbobenzoxy-analogue du GABA ainsi obtenu avec l'acide trifluoroacétique afin d'obtenir le sel trifluoroacétate de l'ester diglycéryl de GABA ou de l'analogue du GABA; et

(e) on forme un sel d'addition avec HCl de l'ester diglycéryl du GABA ou de l'analogue du GABA en faisant réagir le produit de l'étape (d) avec HCl.

70. Le composé de la revendication 1 où $R_2$, $R_3$ and $R_4$ sont des hydrogènes et $R_1$ est vinyle.

71. Le composé de la revendication 70 où A est glucosyl.

72. Le composé de la revendication 70 où A est cholestéryl.

73. Le composé de la revendication 1 que est le triester gamma-vinyl-GABA-di-linolénoyl-glycéryl.

74. Le composé de la revendication 1, qui est le triester di-(gamma-vinyl GABA) linolénoyl-glycéryl.

75. Le composé de la revendication 1 dans lequel A représente le radical de la dihydroxy acétone.